(19) **Europäisches Patentamt / European Patent Office / Office européen des brevets**

(11) **EP 4 382 110 A1**

(12) **EUROPEAN PATENT APPLICATION**
published in accordance with Art. 153(4) EPC

(43) Date of publication:
**12.06.2024 Bulletin 2024/24**

(21) Application number: **22852755.2**

(22) Date of filing: **30.06.2022**

(51) International Patent Classification (IPC):
**A61K 31/713** (2006.01)     **A61P 25/28** (2006.01)
**A61P 35/00** (2006.01)      **A61P 37/02** (2006.01)
**C12N 15/11** (2006.01)

(52) Cooperative Patent Classification (CPC):
**A61K 31/713; A61P 25/28; A61P 35/00;
A61P 37/02; C12N 15/11**

(86) International application number:
**PCT/JP2022/026323**

(87) International publication number:
**WO 2023/013329 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.08.2021 JP 2021128492**

(71) Applicant: **The University of Tokyo
Bunkyo-ku, Tokyo 113-8654 (JP)**

(72) Inventors:
• **OKAMOTO Akimitsu
Tokyo 113-8654 (JP)**
• **MORIHIRO Kunihiko
Tokyo 113-8654 (JP)**

(74) Representative: **Mewburn Ellis LLP
Aurora Building
Counterslip
Bristol BS1 6BX (GB)**

Remarks:
The complete document including Reference Table(s) and the Sequence Listing(s) can be downloaded from the EPO website

(54) **HAIRPIN NUCLEIC ACID COMPOSITION**

(57) The present invention addresses the problem of providing a nucleic acid medicine that is capable of inducing nucleic acid immunity in a cell-specific manner and is capable of capturing and/or inactivating a nucleic acid binding protein for which drug development is difficult. There are provided a hairpin nucleic acid composition that contains two or more kinds of hairpin nucleic acids, and a pharmaceutical composition, a protein function-inhibiting composition, and a cell death-promoting composition that contain the hairpin nucleic acid composition as an active ingredient.

Fig. 1

EP 4 382 110 A1

**Description**

Technical Field

The present invention relates to a hairpin nucleic acid composition, a cell death-inducing composition, and a pharmaceutical composition, a protein function-inhibiting composition, and a cell death-promoting composition that comprise the hairpin nucleic acid composition and the cell death-inducing composition as active ingredients.

Background Art

A nucleic acid medicine is a molecular targeted therapeutic drug based on a nucleic acid molecule that specifically binds to a target nucleic acid or protein to suppress its function. Such nucleic acid medicines have been drawing attention as new medicines against diseases that have been conventionally difficult to treat. In fact, in the 2010s, innovative new drugs have been created in Japan and abroad, such as Givlaari, that target the liver as the site of disease (Non-Patent Literature 1). However, the practical application of nucleic acid medicine, particularly against cancer, has not yet been achieved.

Nucleic acid medicines that have been marketed to date have mainly been antisense nucleic acids and siRNAs, which hybridize with target mRNAs in a sequence-specific manner to inhibit the translation and splicing processes of the mRNAs. In recent years, aptamers and decoy nucleic acids that target proteins have also been intensively studied. In particular, the decoy nucleic acids are capable of capturing and/or inactivating nucleic acid binding proteins, such as transcription factors, for which drug development has been difficult, and are nucleic acid molecules that are promising as anticancer agents. However, there are several problems with the practical application of such decoy nucleic acids as nucleic acid medicines. Primarily, such a decoy nucleic acid has low cell selectivity, and therefore has a high off-target effects and is prone to also exhibit toxicity to a normal cell. In addition, diseases targeted by such decoy nucleic acids are limited to diseases in which target molecules are present.

In conventional drug development for a nucleic acid medicine, the immunotoxicity exhibited by the nucleic acid medicine itself has been a problem. The immunotoxicity is a consequence that a nucleic acid medicine is recognized as a foreign substance by a defense mechanism against a foreign nucleic acid such as a virus (nucleic acid immunity), and a further effort such as chemical modification of a nucleic acid or selection of a sequence is required for avoiding the immunotoxicity (Non-Patent Literature 2).

Until now, efforts have been made to suppress of nucleic acid immunity in nucleic acid medicines. However, induction of nucleic acid immunity against cancer cells and the like in a cell-specific manner may allow an achievement of effective immunotherapy with less side effects. Moreover, the achievement may minimize efforts to examine chemical modifications and sequences for suppressing nucleic acid immunity, and therefore also provides great advantages in production cost and quality control. However, drug development for a nucleic acid medicine from such perspective has rarely been conducted, and there have been no successful examples until now.

Citation List

Non-Patent Literature

Non-Patent Literature 1: Approved Oligonucleotide Therapeutics (May 2021), Section 2, Division of Molecular Target and Gene Therapy Products, National Institute of Health Sciences
Non-Patent Literature 2: Shen, W. et al. Nat. Biotechnol. 2019, 37, 640-650.

Summary of Invention

Technical Problem

The problem to be solved by the present invention is providing a nucleic acid medicine that is capable of inducing nucleic acid immunity in a cell-specific manner and is capable of capturing and/or inactivating a nucleic acid binding protein for which drug development is difficult.

Solution to Problem

To solve the problems described above, the present inventors focused attention on hybridization chain reaction (hereinafter referred to as "HCR" in the present specification) which is one of nucleic acid self-assembly technologies, and has been primarily used in detection of and fluorescent imaging of a slight amount of *in vivo* nucleic acid molecules. The present inventors induced HCR in a cancer cell-specific manner using a hairpin nucleic acid having the protein binding motif of a transcription factor NF-κB, and revealed that the cancer cells died and nucleic acid immunity was induced. The present invention is based on said novel findings and the like, and provides the following.

[1] A hairpin nucleic acid composition comprising two or more kinds of hairpin nucleic acids comprising a whole or part of a protein-binding motif,
wherein the hairpin nucleic acids consist of:

a starting hairpin nucleic acid comprising a target RNA-binding cassette and an elongating hairpin nucleic acid-binding cassette; and
an elongating hairpin nucleic acid comprising a protruding (toehold) region cassette and a non-protruding region cassette;
wherein each hairpin nucleic acid comprises a protruding region with 4 to 20 bases, a stem first region comprising 10 to 20 bases, a loop region with 3 to 50 bases, and a stem second region comprising 10 to 20 bases, in this order;
wherein the stem first region and the stem second region are capable of hybridizing with each other intramolecularly;
wherein in the starting hairpin nucleic acid,
the target RNA-binding cassette comprises a whole or part of the protruding region and a whole or part of the subsequent stem first region, and is capable of hybridizing with a whole or part of a target RNA;
wherein the elongating hairpin nucleic acid-binding cassette comprises a whole or part of the stem second region, and is capable of hybridizing with the protruding region cassette of the elongating hairpin nucleic acid;
wherein in the elongating hairpin nucleic acid,
the protruding region cassette comprises a whole or part of the protruding region and a whole or part of the subsequent stem first region, and is capable of hybridizing with a non-protruding region cassette of another elongating hairpin nucleic acid, and
the non-protruding region cassette comprises a whole or part of the stem second region, and is capable of hybridizing with a protruding region cassette of another elongating hairpin nucleic acid;
wherein the loop region and/or the protruding region comprises a whole or part of the protein-binding motif; and
wherein the target RNA binds to the target RNA-binding cassette of the starting hairpin nucleic acid, and thereby the whole of the protein-binding motif is composed of a double strand.

[2] The hairpin nucleic acid composition according to [1], wherein the starting hairpin nucleic acid comprises a protruding region with 6 to 15 bases.
[3] The hairpin nucleic acid composition according to [1] or [2], wherein the starting hairpin nucleic acid is capable of hybridizing with 6 bases or more of the target RNA.
[4] The hairpin nucleic acid composition according to any of [1] to [3], wherein the elongating hairpin nucleic acid is capable of hybridizing with 6 bases or more of another elongating hairpin nucleic acid.
[5] The hairpin nucleic acid composition according to any of [1] to [4], wherein for the starting hairpin nucleic acid, a free energy change of hairpin structure formation reaction is -20 to -10 kcal/mol.
[6] The hairpin nucleic acid composition according to any of [1] to [5], wherein each of the hybridizable regions consists of the following base sequences:

(1) a base sequence that is completely complementary to a target sequence for hybridizing;
(2) a base sequence comprising the deletion, substitution, or addition of one or plural bases in (1); and
(3) a base sequence that hybridizes with a target sequence for hybridizing under a high stringent condition.

[7] The hairpin nucleic acid composition according to any of [1] to [6], wherein the hairpin nucleic acid comprises DNA and/or RNA nucleotides.
[8] The hairpin nucleic acid composition according to [7], wherein the nucleotides comprise a modified nucleotide.
[9] The hairpin nucleic acid composition according to any one of [1] to [8], comprising plural kinds of the protein-binding motifs.
[10] The hairpin nucleic acid composition according to any of [1] to [9], wherein the protein-binding motif comprises a transcription factor-binding motif.
[11] The hairpin nucleic acid composition according to any of [1] to [10], wherein a protein that is capable of binding to the protein-binding motif comprises an apoptosis-related factor, an autophagy-related factor, or an inflammation-related factor.

[12] The hairpin nucleic acid composition according to any of [1] to [11], wherein the target RNA is mRNA or miRNA.

[13] The hairpin nucleic acid composition according to any of [1] to [12], wherein the target RNA is RNA that is expressed in a cell-specific manner.

[14] The hairpin nucleic acid composition according to [13], wherein the cell comprises any of a cancer cell, an inflammatory cell, or an immune cell.

[15] The hairpin nucleic acid composition according to any of [1] to [14], wherein the target RNA-binding cassette is capable of hybridizing with the non-protruding region cassette of the elongating hairpin nucleic acid.

[16] The hairpin nucleic acid composition according to any of [1] to [15], wherein the elongating hairpin nucleic acid comprises hairpin nucleic acids of 5'-end protruding type and 3'-end protruding type.

[17] A protein function-inhibiting composition comprising the hairpin nucleic acid composition according to any of [1] to [16] as an active ingredient.

[18] A cell death-promoting composition comprising the hairpin nucleic acid composition according to any of [1] to [16] as an active ingredient.

[19] A pharmaceutical composition comprising the hairpin nucleic acid composition according to any of [1] to [16] as an active ingredient.

[20] The pharmaceutical composition according to [19], wherein the pharmaceutical composition is for treating a disease selected from a group consisting of cancers, immune system diseases, and neurodegenerative diseases.

(1) A cell death-inducing composition comprising a hairpin nucleic acid having a hairpin structure capable of forming a hybridization chain structure, wherein the cell death-inducing composition comprises a starting hairpin nucleic acid that hybridizes with a target RNA, and an elongating hairpin nucleic acid that hybridizes with the starting hairpin nucleic acid or another elongating hairpin nucleic acid.

(2) The cell death-inducing composition according to (1), wherein the starting hairpin nucleic acid comprises a target RNA-binding cassette that hybridizes with a target RNA and an elongating hairpin nucleic acid-binding cassette that hybridizes with the elongating hairpin nucleic acid, and the elongating hairpin nucleic acid comprises a protruding region cassette that hybridizes with the starting hairpin nucleic acid or another elongating hairpin nucleic acid, and a non-protruding region cassette that hybridizes with further another elongating hairpin nucleic acid.

(3) The cell death-inducing composition according to (1) or (2), wherein the target RNA binds to the target RNA-binding cassette to allow a hairpin structure of the starting hairpin nucleic acid to be dissociated to enable hybridization between the elongating hairpin nucleic acid-binding cassette and the protruding region cassette of the elongating hairpin nucleic acid, and the hybridization allows a hairpin structure of the elongating hairpin nucleic acid to be dissociated to enable hybridization between the non-protruding region cassette and the protruding region cassette of another elongating hairpin nucleic acid to form a straight-chain double-stranded nucleic acid having the hybridization chain structure.

(4) The cell death-inducing composition according to any of (1) to (3), wherein the elongating hairpin nucleic acid comprises hairpin nucleic acids of 5'-end protruding type and 3'-end protruding type.

(5) The cell death-inducing composition according to any of (1) to (4), wherein the target RNA-binding cassette is capable of further hybridizing with a non-protruding region cassette of the elongating hairpin nucleic acid.

(6) The cell death-inducing composition according to any of (1) to (5), wherein the starting hairpin nucleic acid and/or elongating hairpin nucleic acid further comprise a whole or part of a protein-binding motif.

(7) The cell death-inducing composition according to (6), wherein the loop region and/or the protruding region comprise a whole or part of the protein-binding motif; and the whole of the protein-binding motif comprises a double strand when the hybridization chain structure is formed.

(8) The cell death-inducing composition according to any of (1) to (7), wherein for the starting hairpin nucleic acid, a free energy change of hairpin structure formation reaction is -20 to -10 kcal/mol.

(9) The cell death-inducing composition according to any of (1) to (8), wherein the hairpin nucleic acid comprises DNA and/or RNA nucleotides.

(10) The cell death-inducing composition according to (9), wherein the nucleotides comprise a modified nucleotide.

(11) The cell death-inducing composition according to any of (1) to (10), wherein the target RNA is mRNA or miRNA.

(12) The cell death-inducing composition according to any of (1) to (11), wherein the target RNA is RNA that is expressed or highly expressed in a cell-specific manner.

(13) A pharmaceutical composition comprising the cell death-inducing composition according to any of (1) to (12) as an active ingredient.

(14) The pharmaceutical composition according to (13), wherein the pharmaceutical composition is for treating a disease selected from a group consisting of cancers, immune system diseases, and neurodegenerative diseases.

(15) An anticancer agent comprising a hairpin nucleic acid having a hairpin structure capable of forming a hybridization chain structure, wherein the anticancer agent comprises a starting hairpin nucleic acid that hybridizes with a target RNA, and an elongating hairpin nucleic acid that hybridizes with the starting hairpin nucleic acid or another elongating hairpin nucleic acid.

(16) An anti-inflammatory agent comprising a hairpin nucleic acid having a hairpin structure capable of forming a hybridization chain structure, wherein the anti-inflammatory agent comprises a starting hairpin nucleic acid that hybridizes with a target RNA, and an elongating hairpin nucleic acid that hybridizes with the starting hairpin nucleic acid or another elongating hairpin nucleic acid.

(17) A composition comprising a starting hairpin nucleic acid and an elongating hairpin nucleic acid having a hairpin structure capable of forming a hybridization chain structure, wherein the starting hairpin nucleic acid comprises a target RNA-binding cassette that hybridizes with a target RNA and an elongating hairpin nucleic acid-binding cassette that hybridizes with the elongating hairpin nucleic acid; the elongating hairpin nucleic acid comprises a protruding region cassette that hybridizes with the starting hairpin nucleic acid or another elongating hairpin nucleic acid; and a non-protruding region cassette that hybridizes with further another elongating hairpin nucleic acid; and the starting hairpin nucleic acid and/or the elongating hairpin nucleic acid further comprise a whole or part of a protein-binding motif.

The contents of the disclosures in Japanese Patent Application No. 2021-128492 which forms the basis for priority of the present application are incorporated herein.

Advantageous Effects of Invention

According to the hairpin nucleic acid composition of the present invention, a nucleic acid binding protein can be captured to induce nucleic acid immunity.

According to the protein function inhibitor of the present invention, a protein function can be inhibited based on HCR.

According to the cell death-promoting composition of the present invention, the cell death can be promoted in a cell of interest based on HCR.

According to the pharmaceutical composition of the present invention, an effect based on HCR can be obtained in a site of interest.

Brief Description of Drawings

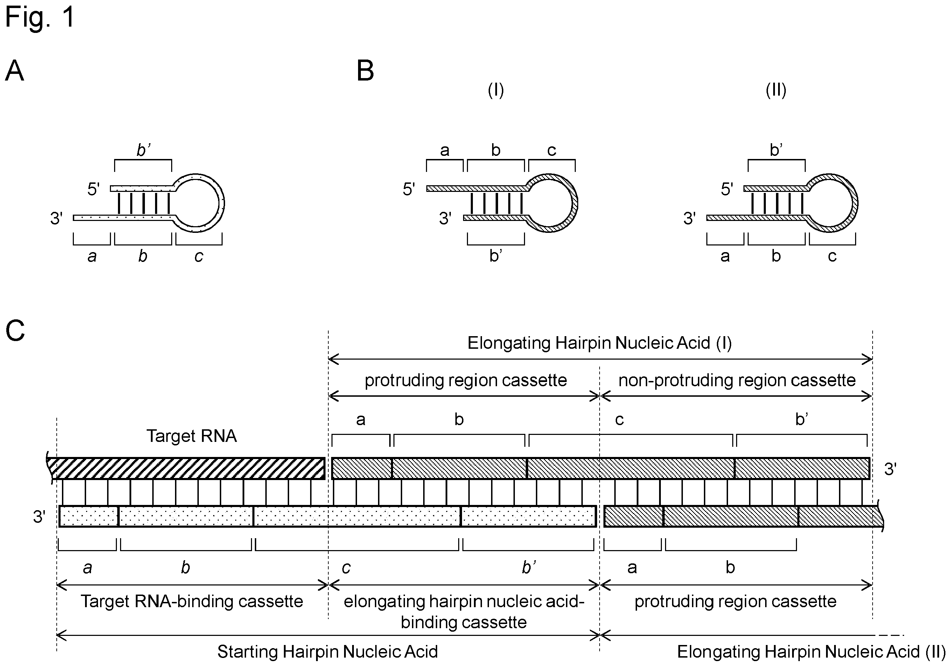

[Figure 1] Figure 1 is a schematic diagram illustrating the typical structure of a hairpin nucleic acid of the present invention. Figure 1A illustrates the structure of a starting hairpin nucleic acid. Figure 1B illustrates the structures of 5'-end ((I)) and 3'-end ((II)) protruding type elongating hairpin nucleic acids. Figure 1C illustrates a state in which a target RNA and the hairpin nucleic acids in Figures 1A and 1B hybridize with each other. In Figure 1, "a" denotes a protruding region, "b" denotes a stem first region, "b'" denotes a stem second region, "c" denotes a loop region, and each thin vertical line in Figure 1C shows the state in which two nucleic acid chains hybridize with each other.

[Figure 2] Figure 2 is a diagram that schematically illustrates a specific example of HCR. Figure 2A illustrates step 1 to step 3 of HCR. Figure 2B illustrates a state in which an HCR product is formed by repeating step 3. In Figure 2, "sHP" represents a starting hairpin nucleic acid, and "eHP" represents an elongating hairpin nucleic acid. In Figure 2A, "Target" represents a target RNA.

[Figure 3] Figure 3 is a diagram indicating HCR efficiency in a set of three hairpin nucleic acids (HP(16), HP(15), and HP(13)) which are different in a minimum free energy change.

[Figure 4] Figure 4 is a diagram indicating the amounts of the existing first hairpin nucleic acid (HP1(16)) and second hairpin nucleic acid (HP2(16)) of HP(16) over time, under the presence of nucleases.

[Figure 5] Figure 5 is a diagram indicating the efficiency of capturing NF-κB by the HCR product of HP(16). In Figure 5, "NF-κB/HCR product" denotes a complex of NF-κB and the HCR product.

[Figure 6] Figure 6 is a diagram indicating a relationship between time after microinjection of HP(16) and the amount of HCR product in HEK293T cells and HeLa cells. In Figure 6, "**" indicates that p-value is less than 0.01.

[Figure 7] Figure 7 is a diagram indicating a relationship between the amount of introduced HP(16) and a cell survival rate in HEK293T cells and HeLa cells. In Figure 7, "**" indicates that p-value is less than 0.01, and "***" indicates that p-value is less than 0.001.

[Figure 8] Figure 8 is a diagram indicating the amounts of IFN-β when poly(dG:dC) and HP(16) are introduced into MCF7 cells, respectively. In Figure 8, "NC" represents negative control.

[Figure 9] Figure 9 is a diagram schematically illustrating a specific example of HCR. Figure 9A illustrates step 1 to step 3 of HCR. Figure 9B illustrates a state in which an HCR product is formed by repeating step 3. In Figure 9, "sHP" represents a starting hairpin nucleic acid, and "eHP" represents an elongating hairpin nucleic acid. In Figure 9A, "Target" represents a target RNA.

[Figure 10] Figure 10 is a diagram indicating the amounts of HCR products after microinjection of HP(16) in HEK293T cells, MDA-MB-231 cells, HeLa cells, and A549 cells. In Figure 10, "*" indicates that p-value is less than 0.05, and "**" represents p-value is less than 0.01. Error bars represent standard deviations.

[Figure 11] Figure 11 is a diagram indicating relative cell survival rates when HP(16) is introduced in HEK293T, MDA-MB-231, and HeLa cells. In Figure 11, "**" indicates that p-value is less than 0.01, and "***" indicates that p-value is less than 0.001. Error bars represent standard deviations.

[Figure 12] Figure 12 is a diagram indicating: relative cell survival rates when poly(dA:dT) and HP(16) into HeLa cells, respectively; and change thereof due to further introduction of STING siRNA. In Figure 12, "****" indicates that p-value is less than 0.0001. Error bars represent standard deviations.

[Figure 13] Figure 13 is a diagram indicating: the amount of mRNA in IFN-β when HP(16) is introduced into HeLa cells; and a change thereof due to further introduction of STING siRNA. In Figure 13, "*" indicates that p-value is less than 0.05. Error bars represent standard deviations.

[Figure 14] Figure 14 is a diagram indicating change in tumor volume over time when PBS, poly(dA:dT), and HP(16) is introduced into mice bearing tumors, respectively. In Figure 14, "**" indicates that p-value is less than 0.01. Error bars represent standard deviations.

Description of Embodiments

1A. Hairpin Nucleic Acid Composition

1A-1. Summary

A first aspect A of the present invention is a hairpin nucleic acid composition containing two or more kinds of hairpin nucleic acids capable of forming a hybridization chain structure. The hairpin nucleic acid composition of the present invention comprises a starting hairpin nucleic acid and an elongating hairpin nucleic acid as essential components, and when the starting hairpin nucleic acid hybridizes with a target RNA, a complex of the target RNA, the starting hairpin nucleic acid, and the elongating hairpin nucleic acid is serially formed by HCR. The hairpin nucleic acid composition of the present invention is capable of capturing a nucleic acid binding protein to induce nucleic acid immunity, and may be an active ingredient of a protein function-inhibiting composition, a cell death-promoting composition, and a pharmaceutical composition of the present invention.

1A-2. Definitions

The following terms frequently used herein are defined as described below.
Herein, "hairpin nucleic acid" refers to a single-stranded nucleic acid that is capable of forming a hairpin structure. Herein, "hairpin structure" refers to the secondary structure of a nucleic acid comprising a set of a stem structure, a loop structure, and a protruding region, formed of single-stranded nucleic acid. Schematic diagrams of hairpin nucleic acids are illustrated in Figures 1A and 1B. The hairpin nucleic acid herein comprises a protruding region (a), a stem first region (b), a loop region (c), and a stem second region (b'), in this order.
Herein, "5'-end protruding type (B (I))" refers to the type of a hairpin nucleic acid comprising a protruding region in the 5' end. The 5'-end protruding type hairpin nucleic acid comprises a protruding region, a stem first region, a loop region, and a stem second region in order from the 5' end. Herein, "3'-end protruding types (A and B (II))" refer to the types of a hairpin nucleic acid comprising a protruding region in the 3' end. The 3'-end protruding type hairpin nucleic acid comprises a stem second region, a loop region, a stem first region, and a protruding region in order from the 5' end.
"Stem structure" is a structure in which two stem regions (b and b') comprising base sequences capable of hybridizing with each other form a double strand.
"Loop structure" is a loop-shaped structure formed of a loop region (c) consisting of a single-stranded nucleic acid.
Herein, "protruding region (a)" refers to a protruding end that recognizes the single-stranded moiety of a target RNA or HCR product in HCR. "Protruding end" refers to a nucleic acid region consisting of a single strand adjacent to either or both of the free ends (ends that is not adjacent to loop region) of a stem region. Herein, the length of the protruding region is not particularly limited, but is, for example, 4 bases or more, 5 bases or more, 6 bases or more, 7 bases or more, or 8 bases or more. Moreover, the protruding region may be made up of, for example, 20 bases or less, 19 bases or less, 18 bases or less, 17 bases or less, 16 bases or less, 15 bases or less, 14 bases or less, 13 bases or less, 12 bases or less, 11 bases or less, 10 bases or less, or 9 bases or less. Specifically, the length of the protruding region may be, for example, 4 to 20 bases.

Herein, "stem regions (b and b')" refer to nucleic acid regions that intramolecularly hybridize with each other to form a stem structure. At least both ends of each stem region consist of bases complementary to each other. The length of each stem region is not particularly limited. For example, the length of each stem region is, 10 bases or more, 11 bases or more, 12 bases or more, 13 bases or more, or 14 bases or more, independently from each other. Moreover, the lengths of the stem regions may be, for example, 20 bases or less, 19 bases or less, 18 bases or less, 17 bases or less, 16 bases or less, or 15 bases or less, independently from each other. Specifically, the lengths of the protruding regions may be, for example, 10 to 20 bases, independently from each other. "Stem first region (b)" refers to a stem region adjacent to the protruding region. Moreover, "stem second region (b')" refers to a stem region that is not adjacent to the protruding region.

Herein, "loop region (c)" refers to a nucleic acid region located between the two stem regions in a single-stranded nucleic acid. The length of the loop region is not particularly limited, but is, for example, 3 bases or more, 4 bases or more, 5 bases or more, 6 bases or more, 7 bases or more, or 8 bases or more. Moreover, the length of the loop region may be, for example, 50 bases or less, 40 bases or less, 30 bases or less, 25 bases or less, 20 bases or less, 19 bases or less, 18 bases or less, 17 bases or less, 16 bases or less, 15 bases or less, 14 bases or less, 13 bases or less, 12 bases or less, 11 bases or less, 10 bases or less, or 9 bases or less. Specifically, the length of the loop region may be, for example, 3 to 50 bases.

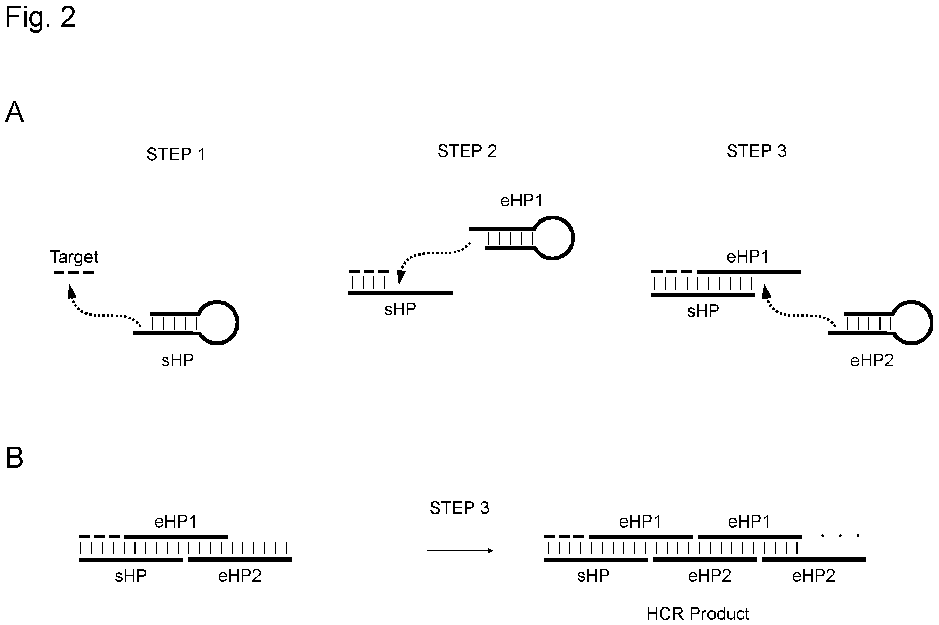

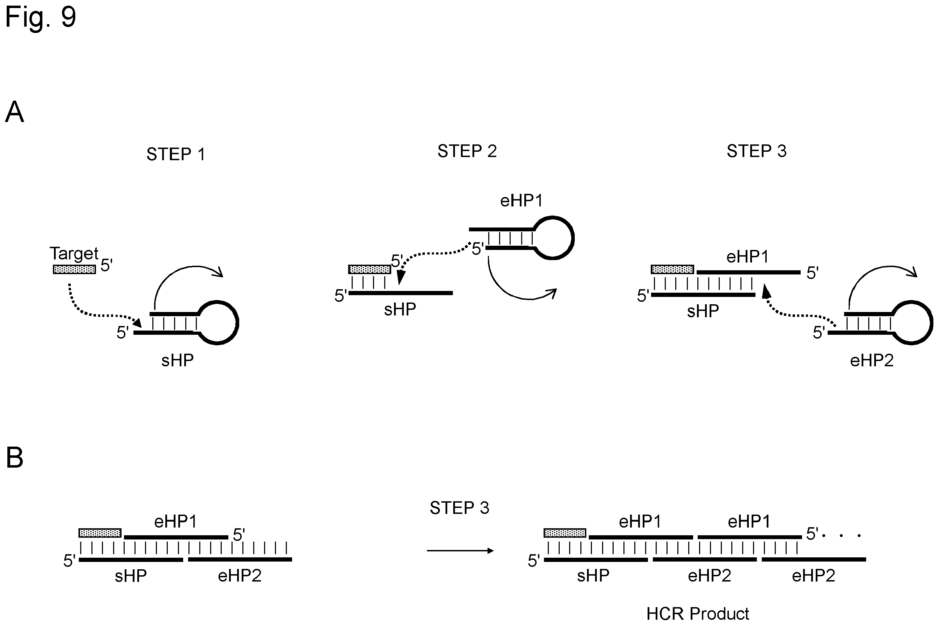

Herein, "hybridization chain reaction ('HCR')" refers to elongation reaction of double-stranded nucleic acid molecules, occurring due to hybridization of a plurality of opened hairpin nucleic acids serially. A process of typical HCR is schematically illustrated in Figures 2 and 9. First, in step 1, a starting hairpin nucleic acid (sHP) recognizes a target RNA with a protruding region, and hybridizes with the target RNA to form a target sequence-starting hairpin nucleic acid complex. In this reaction, the hairpin structure of the starting hairpin nucleic acid is opened by elongation of the hybridization with the target RNA. The formation of the target sequence-starting hairpin nucleic acid complex results in the initiation of the formation of an HCR product. Then, in step 2, the protruding region of a first elongating hairpin nucleic acid (eHP1) recognizes the single-stranded moiety of the starting hairpin nucleic acid in a free state by the opening, and hybridizes with the single-stranded moiety to form a target sequence-starting-elongating hairpin nucleic acid complex. In this reaction, the hairpin structure of the elongating hairpin nucleic acid is opened by elongation of the hybridization with the single-stranded moiety of the starting hairpin nucleic acid. In step 3, a second elongating hairpin nucleic acid (eHP2) recognizes the single-stranded moiety of the elongating hairpin nucleic acid in the target sequence-starting-elongating hairpin nucleic acid complex formed in step 2, is opened, and further hybridizes with the target sequence-starting-elongating hairpin nucleic acid complex to form a target sequence-starting-(elongating)$_2$ hairpin nucleic acid complex. Subsequent repetition of step 3 results in hybridization of elongating hairpin nucleic acids and elongating a double-stranded nucleic acid to form a macromolecular polymer (HCR product) (Figures 2B and 9B). The HCR product forms a straight-chain double-stranded nucleic acid.

Herein, "hybridization chain structure" refers to a nucleic acid structure included in the straight-chain double-stranded nucleic acid formed by the HCR described above. The hybridization chain structure comprises a pathogen-associated molecular pattern, and induces nucleic acid immunity through a pattern-recognition receptor.

Herein, "nucleic acid immunity" refers to a natural immune response based on recognition of a nucleic acid. The nucleic acid immunity is induced by recognition of a non-self nucleic acid and/or damaged autologous nucleic acid by a pattern-recognition receptor.

"Pattern-recognition receptor" is a generic term for a receptor protein which is a protein involved in induction of a natural immune system and recognizes a structure pattern seen in a non-self molecule and/or damaged autologous molecule. Herein, unless otherwise specified, the pattern-recognition receptor refers to a receptor protein that recognizes a nucleic acid molecule. A structure pattern recognized by the pattern-recognition receptor is referred to as "pathogen-associated molecular pattern (PAMP)".

"Pathogen-associated molecular pattern" refers to a molecular pattern that exists in a virus, a prokaryote, and/or a protostome but does not exist in an environment in which a pattern-recognition receptor exists in a normal vertebrate. Herein, "pathogen-associated molecular pattern" particularly refers to a structure pattern that exists in the nucleic acid molecules of a virus, a prokaryote, and/or a protostome but does not exist in the nucleic acid molecule of a vertebrate.

Herein, "target RNA" refers to an RNA initiating the formation of an HCR product by the hairpin nucleic acid composition of the present invention. The target RNA limits conditions under which HCR is induced by the hairpin nucleic acid composition of the present invention. A starting hairpin nucleic acid comprised in a hairpin nucleic acid can specifically hybridize with the target RNA. The kind of the target RNA is not particularly limited. Specifically, for example, the target RNA include mRNAs (including, for example, mature mRNAs, mRNA precursors, and modified mRNAs), non-coding RNAs (ncRNAs: including, for example, micro RNAs (miRNAs), and long non-coding RNAs (lncRNAs)), and natural antisense RNAs.

"Complementary" refers to a relationship in which nucleic acid bases can form base pairing with each other through hydrogen bonds. So-called, Watson-Crick base pairing (natural type base pairing) or Hoogsteen base pairing is included.

"Hybridize" or "hybridizable" represents that base pairing of polynucleotides having base sequences complementary to each other results in the formation of a completely or partly complementary double strand.

Herein, "plural" refers to the number of two or more. Specifically, for example, 2 to 60, 2 to 45, 2 to 30, 2 to 14, 2 to 10, 2 to 8, 2 to 6, 2 to 5, 2 to 4, or 2 to 3.

Herein, "hairpin structure formation reaction" refers to a reaction of a hairpin nucleic acid in which a single-stranded nucleic acid changes from a straight-chain form to a hairpin form to form a hairpin structure.

Herein, "free energy change" refers to the net amount of energy supplied from an external environment to a reaction system through a certain reaction under the condition of constant temperature and pressure. Herein, "free energy change" particularly corresponds to the net amount of energy supplied from the external environment in the hairpin structure formation reaction. For example, when a reaction product is more thermodynamically stable than a starting material, the reaction system losses energy through the reaction, and therefore, the free energy change is negative. The free energy change of the present invention include, for example, both Gibbs' free energy change ($\Delta G$) and Helmholtz's free energy change ($\Delta F$).

Herein, "protein-binding motif" refers to a sequence motif consisting of a double strand to which a specific protein can bind. For example, a protein-binding motif to which a transcription factor binds is referred to herein as "transcription factor-binding motif".

Herein, "protein function-inhibiting" refers to the inhibition of a function that can be originally performed by the protein. The degree of the inhibition and an inhibition method are not particularly limited.

Herein, "cell death" refers to the death of a cell. Such cell deaths are classified roughly into programmed cell deaths and accidental cell deaths. The cell deaths herein include both thereof. The programmed cell death includes, for example, apoptosis, autophagy, and necroptosis. The apoptosis refers to a programmed cell death characterized by formation of an aggregate (apoptotic body) in which a fragmented nucleus is enveloped by a cell membrane. In an apoptosis process, phenomena such as cell fragmentation, nucleus fragmentation, membrane bleb formation, and chromatin condensation are observed. The autophagy herein is also particularly referred to as macroautophagy, and refers to a programmed cell death occurring in nutrient stress. In an autophagy process, a phenomenon, for example, vacuolation in the wide area of cytoplasm is observed. The necroptosis herein refers to a programmed cell death in which a necrosis-like phenomenon such as extracellular release of a cell content is observed. There are several kinds of programmed cell deaths in which such necrosis-like phenomena are observed, but the necroptosis herein encompasses all the programmed cell deaths. The accidental cell death refers to a cell death caused by mechanical damage to a cell or by stress in the exterior or interior of a cell. The accidental cell death is also referred to as necrosis.

Herein, "apoptosis-related factor" refers to an any protein related to a signal pathway that induces apoptosis. The apoptosis-related factor herein includes, for example, both apoptosis-promoting factors and apoptosis-suppressing factors. Specifically, apoptosis-related factor includes, for example, Fas pathway-related factors, caspases, mitochondrial pathway-related factors, FOXO family proteins, and various kinases (for example, cyclin-dependent kinase and MAP kinase). The apoptosis-related factor herein includes, for example, proteins known to be related to diseases such as cancers and neurodegenerative diseases.

Herein, "autophagy-related factor" refers to an any protein related to a signal pathway that induces autophagy. The autophagy-related factor herein includes, for example, both autophagy-promoting factors and autophagy-suppressing factors. The autophagy-related factor may be related to any step of autophagy. The autophagy-related factor includes, for example, factors related to development of phagophore, factors related to growth of phagophore or formation of autophagosome, and factors involved in autolysosome. The autophagy-related factor herein includes, for example, proteins known to be related to diseases such as cancers, immune system diseases, and neurodegenerative diseases.

Herein, "inflammation-related factor" refers to a protein affected during inflammation. The inflammation-related factor herein includes both inflammation-promoting factors and inflammation-suppressing factors. Specifically, the inflammation-related factor includes, for example, various interleukins (including IL-1, TNF-$\alpha$, IFN-$\beta$, and the like), NF-$\kappa$B family proteins, STAT family proteins, and HIF proteins. The inflammation-related factor herein includes, for example, proteins known to be related to diseases such as cancers and immune system diseases.

Herein, "immune system disease" refers to a disease characterized by an abnormality in the immune system. Specifically, the immune system disease includes, for example, autoimmune diseases and inflammatory diseases.

"Immune cell" refers to a cell involved in immunity. Specifically, lymphocytes, macrophages, dendritic cells, and the like are included. For example, the lymphocytes include T cells, B cells, NK cells, and plasma cells. The immune cell herein is preferably a cell having abnormal activity in an immune system disease.

"Autoimmune disease" refers to a disease in which an immune response to a self-antigens is caused. Specifically, for example, Hashimoto's thyroiditis, Graves' disease, lupus, multiple sclerosis, rheumatoid arthritis, hemolytic anemia, systemic lupus erythematosus, celiac disease, Crohn's disease, colitis, type I diabetes mellitus, scleroderma, and psoriasis are included.

"Inflammatory disease" refers to a disease characterized by high-level inflammation or degeneration in a tissue. The inflammatory disease herein includes both chronic inflammatory diseases and acute inflammatory diseases. Specifically, for example, celiac disease, angiitis, lupus, chronic obstructive pulmonary disease (COPD), irritable bowel syndrome, atherosclerosis, arthritis, ankylosing splint, Crohn's disease, colitis, hepatitis (for example, viral hepatitis such as chronic active hepatitis, and non-viral hepatitis), dermatitis, and psoriasis are included. Herein, "inflammatory cell" refers to a cell involved in inflammatory reaction. Specifically, for example, eosinophils, neutrophils, basophils, mast cells, and type II innate lymphocytes are included. The inflammatory cell herein is preferably a cell having abnormal activity in an inflammatory disease.

Herein, "neurodegenerative disease" refers to a disease in which the structure of a nervous tissue is degenerated over time. Specifically, for example, Alzheimer's disease, Parkinson's disease, amyotrophic lateral sclerosis (ALS), Huntington's disease, macular degeneration, multiple sclerosis, muscular dystrophy, Niemann-Pick disease, osteoporosis, and rheumatoid arthritis are included. Most thereof are induced by the accumulation of a specific protein.

Herein, "abnormal activity" refers to more prominent or significant suppression or promotion of activity compared to activity in a normal cell.

Herein, "high expression" of a specific gene refers to a more prominent or significant increase in expression level compared to expression level in a normal cell.

"Statistically significant" means that when statistically analyzing a difference between the measured value of a subject and a control value, a significant difference between then is observed. For example, cases in which the level of significance (significance level) of an obtained value is low, specifically, less than 5% ($p < 0.05$), less than 1% ($p < 0.01$), less than 0.1% ($p < 0.001$), is included. Herein, "p (value)" indicates a probability that a test statistic accidentally agrees with the value in a distribution based on the null hypothesis, in a statistical test. Accordingly, less "p" means that the probability that the test statistic agrees with the value is lower, and the null hypothesis is more easily rejected. The testing method is not particularly limited, and a known testing method by which significance can be assessed may be used as a testing method for the statistical analysis, as appropriate. For example, Student's t-test, covariate-variance analysis, and the like may be used.

1A-3. Components

The hairpin nucleic acid composition of the present invention comprises two or more kinds of hairpin nucleic acids capable of forming a hybridization chain structure, and the hairpin nucleic acid comprises, as an optional component, the whole or part of a protein-binding motif.

The hairpin nucleic acid consists of a starting hairpin nucleic acid and an elongating hairpin nucleic acid. Each thereof will be specifically described below.

(1) Starting Hairpin Nucleic Acid

"Starting hairpin nucleic acid" (Figure 1A) is a hairpin nucleic acid comprising a target RNA-binding cassette and an elongating hairpin nucleic acid-binding cassette (Figure 1C).

"Target RNA-binding cassette" refers to a nucleic acid region that hybridizes with a target RNA in the starting hairpin nucleic acid. Specifically, for example, the target RNA-binding cassette comprises the whole or part of a protruding region and the whole or part of a stem first region following the protruding region (Figure 1C). The target RNA-binding cassette is hybridizable with the whole or part of the target RNA, and, in the base sequence of the cassette, at least both ends of the cassette are complementary to the target RNA.

"Elongating hairpin nucleic acid-binding cassette" refers to a nucleic acid region that hybridizes with an elongating hairpin nucleic acid in the starting hairpin nucleic acid. Specifically, for example, the elongating hairpin nucleic acid-binding cassette comprises the whole or part of a stem second region (Figure 1C). The elongating hairpin nucleic acid-binding cassette is hybridizable with the protruding region cassette of the elongating hairpin nucleic acid, and the base sequences of both the cassettes are complementary to each other in at least both the ends of each of the cassettes.

The starting hairpin nucleic acid may comprise one or plural bases, in addition to the target RNA-binding cassette and the elongating hairpin nucleic acid-binding cassette. The bases may exist, for example, as spacer sequences between the target RNA-binding cassette and the elongating hairpin nucleic acid-binding cassette, or as terminal sequences linked to the free ends of the target RNA-binding cassette and the elongating hairpin nucleic acid-binding cassette, in the starting hairpin nucleic acid.

The cassettes of the starting hairpin nucleic acid may overlap with each other by one or plural bases.

(2) Elongating Hairpin Nucleic Acid

"Elongating hairpin nucleic acid (Figure 1B)" is a hairpin nucleic acid comprising the protruding region cassette and a non-protruding region cassette (Figure 1C).

"Protruding region cassette" refers to a nucleic acid region that hybridizes with the starting hairpin nucleic acid or another elongating hairpin nucleic acid in the elongating hairpin nucleic acid. Specifically, for example, the protruding region cassette comprises the whole or part of the protruding region, and the whole or part of the stem first region following the protruding region (Figure 1C). The protruding region cassette is hybridizable with the elongating hairpin nucleic acid-binding cassette of the starting hairpin nucleic acid or the non-protruding region cassette of another elongating hairpin nucleic acid different from itself, and the base sequences of both the cassettes are complementary to each other in at least both the ends of each of the cassettes.

"Non-protruding region cassette" refers to a nucleic acid region that hybridizes with further another elongating hairpin nucleic acid in the elongating hairpin nucleic acid. Specifically, for example, the non-protruding region cassette comprises the whole or part of the stem second region (Figure 1C). The non-protruding region cassette is hybridizable with the protruding region cassettes of another elongating hairpin nucleic acids different from itself, and the base sequences of both the cassettes are complementary to each other in at least both the ends of each of the cassettes.

Herein, "another elongating hairpin nucleic acid" that hybridizes with the protruding region cassette and "further another elongating hairpin nucleic acid" that hybridizes with the non-protruding region cassette are, usually, elongating hairpin nucleic acid molecules different from each other. However, the elongating hairpin nucleic acid molecules may be elongating hairpin nucleic acids having the same sequence, that is, the same kind of elongating hairpin nucleic acids, or may be elongating hairpin nucleic acids having sequences different from each other, that is, different kinds of elongating hairpin nucleic acids.

The elongating hairpin nucleic acid may comprise one or plural bases, in addition to the protruding region cassette and the non-protruding region cassette. The bases may exist, for example, as spacer sequences between the protruding region cassette and the non-protruding region cassette, or as terminal sequences linked to the free ends of the protruding region cassette and the non-protruding region cassette, in the elongating hairpin nucleic acid.

In both the cassettes of the elongating hairpin nucleic acid may overlap with each other by one or plural bases.

Target RNA ("Target" in Figure 9) binds to the target RNA-binding cassette of the starting hairpin nucleic acid ("sHP" in Figure 9) to dissociate the hairpin structure (particularly, stem structure) of the starting hairpin nucleic acid (steps 1 and 2 in Figure 9A). Then, the elongating hairpin nucleic acid-binding cassette of the starting hairpin nucleic acid and the protruding region cassette of the elongating hairpin nucleic acid ("eHP1" in Figure 9) are enabled to hybridize with each other (step 2 in Figure 9A). The two cassettes hybridize with each other to dissociate the hairpin structure (particularly, stem structure) of the elongating hairpin nucleic acid (steps 2 and 3 in Figure 9A). Then, as in step 2, the non-protruding region cassette of the elongating hairpin nucleic acid and the protruding region cassette of another elongating hairpin nucleic acid ("eHP2" in Figure 9) are enabled to hybridize with each other (step 3 in Figure 9A). A straight-chain double-stranded nucleic acid having a hybridization chain structure is formed by the chain of such a reaction (Figure 9B).

(3) Cassette

The length of each cassette comprised in the hairpin nucleic acid is not particularly limited. The length is, for example, 4 bases or more, 5 bases or more, 6 bases or more, 7 bases or more, 8 bases or more, 9 bases or more, 10 bases or more, 11 bases or more, 12 bases or more, 13 bases or more, 14 bases or more, 15 bases or more, 16 bases or more, 17 bases or more, 18 bases or more, 19 bases or more, 20 bases or more, 21 bases or more, or 22 bases or more. Moreover, the length of each cassette is, for example, 55 bases or less, 50 bases or less, 40 bases or less, 30 bases or less, 25 bases or less, 24 bases or less, or 23 bases or less.

The sequence of each cassette is not particularly limited as long as the sequence comprises a sequence that is hybridizable with a nucleic acid to be hybridized with. For example, each cassette may comprise one or plural bases unrelated to the sequence of a nucleic acid to which the cassette binds.

(3-1) Target RNA-Binding Cassette and Protruding Region Cassette

As described above, each of the target RNA-binding cassette of the starting hairpin nucleic acid and the protruding region cassette of the elongating hairpin nucleic acid preferably comprise the whole or part of the protruding region, and the whole or part of the stem first region following the protruding region. For example, each of the cassettes may be a consecutive region comprising the whole of the protruding region, the stem first region and the loop region, and part of the stem second region, or may be a consecutive region comprising part of the protruding region and part of the stem first region. For example, the sequence of the protruding region, hybridizable with the target RNA or the non-protruding region cassettes of other elongating hairpin nucleic acids different from itself, comprises 4 bases or more, 5 bases or more, 6 bases or more, 7 bases or more, or 8 bases or more. Additionally, for example, the sequence of the stem first region, hybridizable with the target RNA or the non-protruding region cassettes of other elongating hairpin nucleic acids different from itself, comprises 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 14 bases or more, 15 bases or more, or 16 bases or more.

(3-2) Elongating Hairpin Nucleic Acid-Binding Cassette and Non-Protruding Region Cassette

As described above, each of the elongating hairpin nucleic acid-binding cassette of the starting hairpin nucleic acid and the non-protruding region cassette of the elongating hairpin nucleic acid preferably comprise the whole or part of the stem second region. For example, each of the cassettes may be a consecutive region comprising part of the stem first region, the whole of the loop region, and the whole of the stem second region, or may be a consecutive region comprising only part of the stem second region. Moreover, when the hairpin nucleic acid comprises a protruding end adjacent to the stem second region, the cassettes may comprise the whole or part of the protruding end. Specifically, for example, the sequence of the stem second region, hybridizable with the non-protruding region cassette of the elongating hairpin nucleic acid or the protruding region cassette of another elongating hairpin nucleic acids different from itself, comprises 4 bases or more, 5 bases or more, 6 bases or more, 7 bases or more, 8 bases or more, 9 bases or more, 10 bases or more, 11 bases or more, 12 bases or more, 13 bases or more, 14 bases or more, 15 bases or more, or 16 bases or more.

(4) Hybridization Chain Structure

The hairpin nucleic acid of the present invention forms a straight-chain double-stranded nucleic acid having a hybridization chain structure in the presence of the target RNA. The hybridization chain structure can induce nucleic acid immunity.

The specific structure of the hybridization chain structure is not particularly limited as long as the structure is the double-strand structure of a nucleic acid capable of inducing nucleic acid immunity. For example, the hybridization chain structure comprises a pathogen-associated molecular pattern that is recognized by a pattern-recognition receptor existing on a biological membrane or in the cytoplasm.

The pattern-recognition receptor capable of recognizing a nucleic acid existing on the biomembrane includes, for example, Toll-like receptors such as TLR3, TLR7, TLR8, and TLR9. Such receptors exist on the biological membranes of endosome and lysosome, and recognize a nucleic acid molecule entering a cell.

The pattern-recognition receptor capable of recognizing a nucleic acid existing in the cytoplasm includes, for example, RIG-I-like receptors (for example, RIG-I, MDA5, LGP2, and the like), cGAS, and AIM2.

The pathogen-associated molecular pattern that is recognized by each pattern-recognition receptor is known in the art. For example, TLR3 is known to recognize a double-stranded RNA of 40 base pairs or more, and TLR7 and TLR8 are known to recognize, e.g., a polyuracil, a double-stranded RNA rich in guanine and uracil. Moreover, TLR9 is known to recognize, e.g., an unmethylated single-stranded DNA comprising a sequence rich in cytosine and guanine, such as 5'-GTCGTT-3'. Further, the RIG-I protein is known to recognize, e.g., a double-stranded RNA comprising a blunt end and having a triphosphate in the 5' end. In addition, the AIM2 protein is known to recognize, e.g., a double-stranded DNA.

For example, the hybridization chain structure may have a pathogen-associated molecular pattern that is recognized by the pattern-recognition receptor existing in cytoplasm, or may have a pathogen-associated molecular pattern that is recognized by the pattern-recognition receptor that recognizes a double-stranded nucleic acid (for example, double-stranded DNA and/or double-stranded RNA). For example, the pathogen-associated molecular pattern may be a double-strand structure that is recognized by cGAS, or may be a double-strand structure that is recognized by MDA5.

cGAS (cyclic GMP-AMP synthase) is a cyclic GMP-AMP synthase. The exemplary amino acid sequence of cGAS is shown in SEQ ID NO: 14. cGAS bound to a foreign double-stranded nucleic acid (for example, nucleic acid derived from virus) or self abnormal double-stranded nucleic acid (for example, nucleic acid leaked from nucleus in senescent cell or the like) synthesizes 2'-5'-cGAMP, which is a second messenger, from GTP and ATP, to activate a downstream STING (stimulator of interferon genes) pathway through the 2'-5'-cGAMP. The activated STING pathway induces cell apoptosis and the like.

Known examples of nucleic acids that are recognized by cGAS include a long double-stranded DNA that is not included in a chromatin structure, and a short double-stranded DNA having an end comprising guanosine of which is not paired. The immune response pathway caused by cGAS is known to likely be induced, e.g., when a large amount of nucleic acid molecules enter a cell, or when a double-stranded nucleic acid comprising an oxidated DNA molecule enters a cell.

MDA5 (melanoma differentiation-associated gene 5) is a pattern-recognition receptor in the cytoplasm, belonging to a RIG-I family. The exemplary amino acid sequence of MDA5 is shown in SEQ ID NO: 15. MDA5 bound to a double-stranded nucleic acid binds to MAVS (mitochondrial antiviral signaling protein) existing on a mitochondrial outer membrane, and activates a MAVS pathway. The activated MAVS pathway induces an immune response through type I interferon or the like.

The nucleic acid that is recognized by MDA5 includes double-stranded RNA. In particular, a long double-stranded RNA and poly(I:C) molecule are included.

For example, the hybridization chain structure has a certain length. Specifically, the median of the lengths of formed HCR products may be, for example, 100 base pairs or more, 150 base pairs or more, 200 base pairs or more, 250 base pairs or more, 300 base pairs or more, 350 base pairs or more, 400 base pairs or more, 450 base pairs or more, 500 base pairs or more, or 550 base pairs or more. It is preferable to form a constant amount of double-strand structure of 500 base pairs or more. For example, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, or 25% or more of the HCR products have a double-strand structure of 500 base pairs or more.

(5) Protein-Binding Motif

The loop region and/or the protruding region comprise the whole or part of the protein-binding motif, as an optional component. In such a case, the whole of the protein-binding motif is composed of a double strand by binding of the target RNA to the target RNA-binding cassette of the starting hairpin nucleic acid. In the state of the hairpin nucleic acid, the whole or part of the protein-binding motif is in the state of a single strand, and the HCR causes the entire of protein-binding motif to be double stranded. For example, the whole or part of each of one or plural sequence motifs may be comprised in one molecule, or the complete sequence motif need not be included in one molecule. In the case of including a plurality of motifs, the kinds of the motifs are not particularly limited. For example, all of the plurality of sequence motifs may be the same, or may include more than one kind of sequence motifs. A protein capable of binding to a double-stranded nucleic acid and the binding motif thereof may be determined by a known method. Known databases such as Transfac, WordSpy, T-Reg Comparator, MOTIF, TFBIND, TFSEACH, and JASPAR may be used. For example, the protein-binding motif may be identified by DNA footprinting, gel mobility shift assay, or another known method, and/or may be predicted based on a known consensus sequence motif.

The number of proteins that bind to each sequence motif is not particularly limited. For example, a sequence motif to which one or plural kinds of proteins can bind may be used. The kind of binding protein is not particularly limited. For example, transcription factors and polymerases are included. The protein is preferably a transcription factor. In such a case, the protein-binding motif comprises a transcription factor-binding motif. The protein may be, for example, a protein involved in a specific disease. Specifically, for example, the protein comprises an apoptosis-related factor, an autophagy-related factor, an inflammation-related factor, and the like.

(6) Hairpin Nucleic Acid Composition

Each hairpin nucleic acid comprised in the hairpin nucleic acid composition of the present invention may be composed of DNA and/or RNA nucleotides. Each hairpin nucleic acid may comprise a modified nucleotide as a composing nucleotide. The kind, number, position, and the like of the comprised modified nucleotide are not particularly limited. Specifically, for example, modifications of internucleotide bonds, analogs having properties and/or structures similar to those of DNA and RNA, and peptide nucleic acids are included. Although a selection of a modification may depend on the sequence of a target RNA, or the like, those skilled in the art may determine a preferred embodiment with reference to the description of literature related to a nucleic acid medicine (for example, WO 2007/143315). Moreover, an objective of the modification is not particularly limited. For example, the modification may be carried out for stabilizing and detecting the hairpin nucleic acid and the HCR product, and for performance of the pharmacology functions of the hairpin nucleic acid and the HCR product.

The starting hairpin nucleic acid and the elongating hairpin nucleic acid comprised in the hairpin nucleic acid composition may comprise plural kinds of hairpin nucleic acids.

Each of the starting hairpin nucleic acid and the elongating hairpin nucleic acid comprised in the hairpin nucleic acid composition of the present invention may be any of 5'-end protruding types and 3'-end protruding types. Moreover, the numbers of the 5'-end protruding types and the 3'-end protruding types in the starting hairpin nucleic acid and the elongating hairpin nucleic acid are not particularly limited. The elongating hairpin nucleic acid preferably comprises both the 5'-end protruding type and the 3'-end protruding type.

The free energy change of hairpin structure formation reaction is not particularly limited. Usually, when the value of the free energy change of the hairpin structure formation reaction is lower, a hairpin structure is hardly opened, while the value of the free energy change of the hairpin structure formation reaction results in the hairpin structure being more easily opened. The free energy change is, for example, -20 to -10 kcal/mol. Specifically, the free energy change is, for example, -20 kcal/mol or less, -19 kcal/mol or less, -18 kcal/mol or less, -17.5 kcal/mol or less, -17 kcal/mol or less, or -16.5 kcal/mol or less. Moreover, the free energy change is, for example, -10 kcal/mol or more, -11 kcal/mol or more, -12 kcal/mol or more, -12.5 kcal/mol or more, -13 kcal/mol or more, -13.5 kcal/mol or more, -14 kcal/mol or more, -14.5 kcal/mol or more, or -15 kcal/mol or more. The free energy change may be determined using known software that is used in the prediction of the stability of the higher-order structure of a nucleic acid, such as NUPAK.

The lengths of the regions and the free energy change in each hairpin nucleic acid need not be equal to each other. Usually, the opening reaction of the hairpin structure of the hairpin nucleic acid that is most hardly opened, among hairpin nucleic acids involved in a series of HCR processes, is considered to be a rate-determining step of elongation reaction of HCR. For example, the free energy change of the hairpin structure formation reaction of the starting hairpin nucleic acid may be less than the free energy change of the elongating hairpin nucleic acid.

The starting hairpin nucleic acid is a hairpin nucleic acid that is hybridizable with the target RNA, and the elongating hairpin nucleic acid is a hairpin nucleic acid that is hybridizable with another hairpin nucleic acid. The starting hairpin nucleic acid and the elongating hairpin nucleic acid are names based on the functions thereof, and therefore, one hairpin nucleic acid may have the functions as both the starting hairpin nucleic acid and the elongating hairpin nucleic acid. For example, when plural kinds of starting hairpin nucleic acids are comprised, all the starting hairpin nucleic acids may be hybridizable with one kind of elongating hairpin nucleic acid, or may be hybridizable with different elongating hairpin nucleic acids.

Preferably, the starting hairpin nucleic acid is hybridizable with one or more elongating hairpin nucleic acids with the different end protruding type from itself. For example, when the starting hairpin nucleic acid is a 5'-end protruding type, at least one of 3'-end protruding type elongating hairpin nucleic acids may be hybridizable with the starting hairpin nucleic acid. Preferably, all the elongating hairpin nucleic acids are hybridizable with two or more elongating hairpin nucleic acids with the different end protruding type from itself.

The whole or part of each cassette included in the hairpin nucleic acid may be hybridizable with the whole or part of plural kinds of target RNAs or the cassettes of plural kinds of hairpin nucleic acids. For example, when one hairpin nucleic acid has the functions as both the starting hairpin nucleic acid and the elongating hairpin nucleic acid, the whole or part of the target RNA-binding cassette of the hairpin nucleic acid is hybridizable with the non-protruding region cassette of the elongating hairpin nucleic acid. In such a case, parts of the target RNA-binding cassette and the protruding region cassette overlap with each other. For example, when the target RNA-binding cassette and the protruding region cassette correspond to each other, the target RNA-binding cassette is hybridizable with the non-protruding region cassette of the elongating hairpin nucleic acid. Moreover, the whole or part of the cassettes of plural kinds of hairpin nucleic acids may be hybridizable with the whole or part of the same cassette or the target RNA.

The manner of the hybridization between the cassettes is not particularly limited. Preferably, when the cassettes hybridize with each other, the other cassettes of each hairpin nucleic acid are exposed to different directions opposite to the hybridized cassettes. For example, when the elongating hairpin nucleic acid-binding cassette of the starting hairpin nucleic acid and the protruding region cassette of the elongating hairpin nucleic acid hybridize with each other, a double-stranded region is flanked between two cassettes exposed as a single strand (target RNA-binding cassette of starting hairpin nucleic acid and non-protruding region cassette of elongating hairpin nucleic acid), in a complex of the hairpin nucleic acid. Therefore, cassettes on the side of 3'-end may be hybridizable with each other, and cassettes on the side of 5'-end may be hybridizable with each other. The manner of the hybridization between the starting hairpin nucleic acid and the target RNA is not particularly limited.

The base sequence of a hybridizable region is not particularly limited as long as the base sequence is hybridizable. The specific hybridizable base sequence consists of, for example, the following sequences:

(1) a base sequence completely complementary to a target sequence for hybridizing;
(2) a base sequence comprising the deletion, substitution, or addition of one or plural bases in (1); and
(3) a base sequence that hybridizes with the target sequence for hybridizing under a high stringent condition.

Hybridizability may be determined using a method known in the art. For example, such hybridizability may be determined based on base identity. Usually, a second nucleic acid is hybridizable with the first nucleic acid when the base identity of the base sequence of the second nucleic acid to a sequence completely complementary to the base sequence of the first nucleic acid is not less than a certain level. Specifically, for example, when the base identity is 70% or more, 80% or more, 90% or more, 95% or more, 96% or more, 97% or more, 98% or more, 99% or more, or 100%, nucleic acids are hybridizable. Herein, "base identity" refers to the percentage (%) of the identical bases of one polynucleotide with respect to the number of all the bases of the other polynucleotide when the base sequences of the two polynucleotides are aligned (subjected to alignment), and, as needed, a gap is introduced into either of the base sequences to allow the degree of the coincidence of the bases of both the base sequences to be the highest. % identity may be easily determined using a known program such as a homology search program BLAST (Basic local alignment search tool, Altschul, S. F. et al, J. Mol. Biol., 215, 403-410, 1990) search. Usually, the second nucleic acid having a base sequence comprising substitution of a plurality of bases with other bases in a sequence completely complementary to the base sequence of the first nucleic acid is hybridizable with the first nucleic acid. Specifically, for example, when 2 to 60, 2 to 45, 2 to 30, 2 to 14, 2 to 12, 2 to 10, for example, 2 to 8, 2 to 6, 2 to 5, 2 to 4, or 2 to 3 bases are substituted, nucleic acids are hybridizable.

Hybridization conditions are not particularly limited but may be, for example, various stringent conditions such as low and high stringent conditions. "Low stringent condition" means a condition under which nucleic acids hybridize easily. Such low stringent conditions refer to conditions of low temperature and high salt concentration in washing after hybridization. For example, the low stringent conditions are conditions of performing washing at 42°C to 50°C using, for example, a buffer comprising 5× SSC and 0.1% SDS in washing after hybridization. "High stringent condition" means a condition under which a nonspecific hybrid is not formed. Low salt concentration herein specifically refers to, for example, 15 to 750 mM, preferably, 15 to 500 mM, 15 to 300 mM, or 15 to 200 mM. High temperature herein is, specifically, for example, 50 to 68°C, or 55 to 70°C. Specific examples of high stringent conditions include conditions of washing at 65°C and with 0.1× SSC, and 0.1% SDS. Herein, the 1× SSC comprises 150 mM sodium chloride and 15 mM sodium citrate.

The manner of expression of the target RNA is not particularly limited. For example, the target RNA may be systemically expressed regardless of timing and the presence or absence of a stimulation, or may be expressed in a tissue-specific, cell-specific, or timing-specific manner, or in response to a stimulation such as an extracellular signal. The target RNA is preferably an RNA that is expressed in a cell-specific manner. In such a case, a cell in which the target RNA is expressed is not particularly limited. Specifically, the cell is, for example, a cancer cell, inflammatory cell, or immune cell. The inflammatory cells and immune cells in which the target RNA is expressed preferably exhibit abnormal activity.

The manner of expression of a protein that binds to the hybridization chain structure and the protein-binding motif is not particularly limited. For example, the protein may be systemically expressed regardless of timing and the presence or absence of stimulation, or may be expressed in a tissue-specific, cell-specific, or timing-specific manner, or in response to a stimulation such as an extracellular signal.

It is not necessary that the protein is capable of stably binding to the sequence motif under any conditions. The protein may be capable of binding to the sequence motif under a specific condition. For example, the protein of the present invention includes proteins such as a methylated CpG binding protein, whose binding does not occur by the sequence itself of the sequence motif but occurs only when the sequence motif comprises a specific modification. It is not necessary that a condition under which the protein is capable of binding to the sequence motif is satisfied at the time of production and use of the hairpin nucleic acid composition of the present invention, and may be satisfied, for example, through intracellular reaction.

1A-4. Effects

The hairpin nucleic acid composition of the present invention is capable of binding to a specific protein through the hybridization chain structure formed by inducing HCR in the presence of the target RNA, or further through the protein-binding motif existing in the state of a double strand in an HCR product. The HCR product becomes, for example, a polymer having a size of around 100 bp to 1000 bp, and a protein such as a pattern-recognition receptor binds to the polymer depending on the length and structure of the hybridization chain structure. When protein-binding motifs are included, a large number of specific proteins further bind to the motifs depending on the number of the protein-binding motifs. As a result, for example, an aggregate is formed, and liquid-liquid phase separation is induced to enable stress on a cell to be applied. Herein, the liquid-liquid phase separation is also referred to as biological phase separation, and means that a mixed system that is uniformly mixed is changed from a one-phase liquid state to a liquid state with two phases which can be distinguished, due to a change in condition.

Nucleic acid immunity is induced by the hairpin nucleic acid composition of the present invention. The nucleic acid immunity refers to an immune response based on the recognition of a nucleic acid by the innate immune system. The nucleic acid immunity is an immune response induced by viral infection, and activates the transcription factor of an interferon regulatory factor (IRF) family on the basis of recognition of a nucleic acid by a pattern-recognition receptor or the like including a Toll-like receptor or the like. In such a case, expression of type I interferons (INF-$\alpha$, INF-$\beta$) and the like may be induced. Alternatively, for example, capture of a specific protein existing in an excessive amount enables the function of a protein to be inhibited, and the amount of free protein to be decreased. As a result, for example, the function of a protein that protects a cancer cell from the immune system (for example, NF-$\kappa$B signal pathway-related protein) may be inhibited.

Such effects may be measured by, for example, administering a hairpin nucleic acid composition to be tested to a subject (for example, an experimental animal, a cultured cell, or the like), and measuring the indices of the effects, produced by the hairpin nucleic acid composition to be tested, after a certain period, for example, 30 minutes, 1 hour, 2 hours, 3 hours, 6 hours, 12 hours, 1 day, or several days (for example, 1 to 7 days, or 2 to 7 days) after the administration. Specifically, the measurement may be performed by, for example, measuring the survival rate of the cells, the index of cell stress, such as a cell stress marker or a cell death marker, or the like, for example, for the purpose of applying stress on cells. For example, in the case of capturing a protein, the measurement may be performed by, for example, measuring the amount of the free protein thereof, or the amount of a reaction product related to the function of the protein (for example, expression of genes for which transcription is regulated, product of catalyzed enzyme reaction, activity of mediated signal pathway, or the like).

For example, an index, or the level of a product, expected to be decreased by applying the hairpin nucleic acid, may be decreased compared to the negative control (for example, administration of vehicle), or a level such as an index expected to be increased may be increased compared to the negative control. An amount of change in the level in such a case is not particularly limited. Specifically, for example, a change of at least 10%, at least 20%, at least 25%, at least 30%, or at least 40% shows that a hairpin nucleic acid composition to be tested can produce an effect of interest. Alternatively, for example, whether an effect of interest can be produced may be determined based on whether the change is statistically significant.

1B. Cell Death-Inducing Composition

1B-1. Summary

A first aspect B of the present invention is a cell death-inducing composition. The cell death-inducing composition of the present invention comprises a starting hairpin nucleic acid and an elongating hairpin nucleic acid, having a hairpin structure capable of forming a hybridization chain structure, as active ingredients. When the starting hairpin nucleic acid hybridizes with a target RNA, a complex of the target RNA, the starting hairpin nucleic acid, and the elongating hairpin nucleic acid is formed serially by HCR. The cell death-inducing composition of the present invention can capture a nucleic acid binding protein to induce nucleic acid immunity, and can be the active ingredient of the cell death-promoting composition, pharmaceutical composition, anticancer agent, anti-inflammatory agent, or nucleic acid immunity-inducing composition of the present invention.

1B-2. Components

The components of the cell death-inducing composition of the present aspect will be described. The cell death-inducing composition of the present invention comprises the starting hairpin nucleic acid and the elongating hairpin nucleic acid as essential components, and a carrier as an optionally selectable component. Each component is specifically described below.

The starting hairpin nucleic acid and the elongating hairpin nucleic acid are as described in detail in the first aspect A. Accordingly, detailed descriptions thereof are omitted.

The cell death-inducing composition of the present aspect comprises effective amounts of the starting hairpin nucleic acid and the elongating hairpin nucleic acid. Basically, the details of the active ingredients are in accordance with those of a pharmaceutical composition described in a second aspect described later except that a desired effect is induction of cell death (for example, apoptosis). Accordingly, differences will only be described.

The cell death-inducing composition of the present invention is intended to induce cell death. Accordingly, the cell death-inducing composition may comprise one or more active ingredients capable of inducing cell death as active ingredients, in addition to the hairpin nucleic acid composition described in the first aspect A. Specific examples of the active ingredients include compounds, drugs based on the promotion or suppression of gene expression, and drugs based on promotion or inhibition of functions at protein levels, that induce cell death.

It is not necessary that the cell death-inducing composition of the present invention induces cell death only in a cell comprising an HCR product. For example, when the hybridization chain structure is a structure that is recognized by cGAS, 2'-5'-cGAMP, which is a second messenger synthesized by cGAS, may migrate to adjacent cells and the like through gap junctions to also induce cell death in those cells. For example, the cell death of a cell comprising an HCR product may result in the induction of an immune response. The immune response may result in the promotion of secondary cell death (for example, immunogenic cell death) in a cell that does not comprise any HCR product but has a similar abnormality.

2. Pharmaceutical Composition

2-1. Summary

The second aspect of the present invention is a pharmaceutical composition. The pharmaceutical composition of the present invention comprises the hairpin nucleic acid composition and/or cell death-inducing composition described in the first aspect, as active ingredients, and induces HCR in a site of interest, comprising a target RNA. A desired effect based on HCR can be obtained in the site of interest using the pharmaceutical composition of the present invention.

2-2. Components

The components of the pharmaceutical composition of the present aspect are described. The pharmaceutical composition of the present invention comprises active ingredients as essential components, and a carrier as an optionally selectable component. Each component will be specifically described below.

(1) Active Ingredients

The pharmaceutical composition of the present invention comprises effective amounts of the hairpin nucleic acid composition and/or cell death-inducing composition described in the first aspect, as the essential active ingredients. Since the components of the hairpin nucleic acid composition and the cell death-inducing composition are described in detail in the first aspect, specific descriptions thereof are omitted. One or more additional active ingredients may be comprised depending on a desired effect achieved by the pharmaceutical composition.
"Effective amounts" refer to amounts conferring a subject to which the composition is applied little or no harmful side effect and required for performing the functions of the hairpin nucleic acid composition and/or the cell death-inducing composition as the active ingredients. The effective amounts may be changed by various conditions such as information on the subject, the route of administration, and the number of doses. The final decision is made by discretion of a person who performs the administration, including a doctor, a veterinarian, or a pharmacist.
For example, when the hybridization chain structure comprises a structure that is recognized by cGAS, the effective amounts may be determined so that the hybridization chain structure in an amount sufficient for activating cGAS is formed in a cell. Specifically, for example, the effective amounts may be set so that the amount of a nucleic acid in a target cell is 0.01 nM to 20 nM, 0.05 nM to 15 nM, 0.08 nM to 12 nM, 0.09 nM to 11 nM, 0.1 nM to 10 nM, 0.11 nM to 10 nM, 0.2 nM to 10 nM, 0.5 nM to 10 nM, 0.8 nM to 10 nM, 0.9 nM to 10 nM, 1 nM to 10 nM, 2 nM to 10 nM, 5 nM to 10 nM, 0.1 nM to 10 nM, 0.1 nM to 9 nM, 0.1 nM to 8 nM, 0.1 nM to 6 nM, 0.1 nM to 5 nM, 0.1 nM to 3 nM, 0.1 nM to 2 nM, 0.1 nM to 1 nM, 0.2 nM to 0.9 nM, or 0.3 nM to 0.8 nM, without particular limitation.
Herein, "subject" refers to a subject to which the hairpin nucleic acid composition, cell death-inducing composition, pharmaceutical composition, protein function-inhibiting composition, cell death-promoting composition, and nucleic acid immunity induction composition of the present invention are applied. The subject may include an organ, a tissue, and a cell, as well as an individual. The subject may be any animal including a human when being an individual. For example, the animal includes various domestic animals, poultry, companion animals, and experimental animals, as well as humans. Without limitation, the subject may be an individual having abnormal expression of a protein, or an abnormal cell, or may be an individual requiring treatment or prevention of a disease.
Herein, "information on subject" refers to various information of individual on a living body to which application is performed. When the subject is a human, the information includes, for example, age, body weight, gender, dietary habit, health condition, the degree of progression and severity of a disease, drug sensitivity, and the presence or absence of a concomitant drug.
Herein, "site of interest" refers to a site in the living body comprising a cell of interest. Herein, "cell of interest" refers to a cell presumed to exert an effect by the hairpin nucleic acid composition and/or cell death-inducing composition of the present invention. Specifically, the cell of interest is a cell that expresses a target RNA and/or a cell that highly expresses the target RNA. For example, the cell of interest may be a cell that further expresses a pattern-recognition receptor, or may be a cell that further expresses a protein capable of binding to a protein-binding motif.

(2) Carrier

The pharmaceutical composition of the present invention may comprise a pharmaceutically acceptable carrier. "Pharmaceutically acceptable carrier" refers to an additive that is commonly used in the technical field of pharmaceutical preparation. For example, solvents, bases, emulsifiers, suspending agents, surfactants, pH adjusters, stabilizing agents, flavoring agents, excipients, vehicles, antiseptic agents, binders, diluents, isotonizing agents, sedatives, buffer agents, coating agents, lubricants, coloring agents, thickeners, dissolution auxiliaries, and other additives are included.
A solvent may be, for example, water or another pharmaceutically acceptable aqueous solution, or a pharmaceutically acceptable organic solvent (for example, vegetable oil or the like). An aqueous solution includes, for example, physiological saline, isocratic liquids comprising glucose, and other auxiliary agents, phosphate buffers, and sodium acetate buffers. The auxiliary agent includes, for example, nonionic surfactants having low concentration, and polyoxyethylene sorbitan fatty acid esters, as well as D-sorbitol, D-mannose, D-mannitol, and sodium chloride.
The carrier are used to avoid or suppress *in vivo* decomposition of the hairpin nucleic acid composition and/or the cell death-inducing composition, which are active ingredients, caused by an enzyme and/or the like, as well as to facilitate formulation and an administration method, and to maintain dosage form and drug efficacy, and may be used as appropriate, as needed.

(3) Dosage Form

The dosage form of the pharmaceutical composition of the present invention is not particularly limited as long as being a form in which the hairpin nucleic acid composition and/or the cell death-inducing composition, which are active ingredients, according to the first aspect can be delivered to a target site without being inactivated by decomposition and/or the like, and the pharmacological effects of the active ingredients can be produced *in vivo.*

The specific dosage form depends on an administration form and/or a prescription condition. The administration form of the pharmaceutical composition of the present invention may be classified roughly into oral administration and parenteral administration. When the administration method is parenteral administration, a preferred dosage form is a liquid that can be directly administered to a target site or systemically administered through the circulating system. Examples of the liquid include injectable agents. An injectable agent may be formulated in combination with the excipient, an elixir, an emulsifier, a suspension, a surfactant, a stabilizer, a pH regulator, or the like as appropriate, and mixing the resultant in a unit dosage form required for performing commonly approved medicine manufacture. In addition, the dosage form may be an ointment, a plaster, a cataplasm, a transdermal agent, a lotion, an inhalant, an aerosol, an ophthalmic solution, or a suppository.

Both the specific shape and size of each of the dosage forms described above may be within the ranges of the dosage form known in the art, and are not particularly limited. With regard to a method of producing the pharmaceutical composition of the present invention, formulation may be performed according to a common method in the art.

The hairpin nucleic acid composition and/or the cell death-inducing composition of the present invention are/is excellent in solubility in water, Japanese Pharmacopeia second fluid for dissolution test, or Japanese Pharmacopeia second fluid for disintegration test, and have properties excellent for a pharmaceutical product, for example, are excellent in pharmacokinetics (for example, drug half-life in blood, intracerebral transferability, metabolic stability, and CYP inhibition), have low toxicity (for example, superiority as a medicine in view of acute toxicity, chronic toxicity, genetic toxicity, genotoxicity, cardiotoxicity, drug interaction, carcinogenicity, phototoxicity, or the like), and also have fewer side effects (for example, suppression of sedation, or avoidance of laminar necrosis).

(4) Administration Style and Dose

Herein, a preferred style of administration of the pharmaceutical composition of the present invention is not specifically limited. For example, the style may be oral administration or parenteral administration. The parenteral administration is usually used.

Specific examples of the parenteral administration include intramuscular administration, intravenous administration, intraarterial administration, intraperitoneal administration, subcutaneous administration (including implant-type continuous subcutaneous administration), intradermal administration, trachea/bronchus administration, rectal administration, administration via blood transfusion, intratumoral administration, peritumoral administration (for example, intradermal administration or subcutaneous administration at peritumoral site), intracerebroventricular administration, intrathecal administration, nasal administration, and intramuscular administration.

Even repetitive administration of the pharmaceutical composition of the present invention additively produces a suppressive effect in a cell. In the case of the repetitive administration, efficacy may be improved at an administration interval to a certain degree (for example, half a day or more).

(5) Target Diseases

Diseases to which the pharmaceutical composition of the present invention is applied are not particularly limited. For example, the pharmaceutical composition may be applied to diseases with the abnormal expression of a specific protein or the presence of an abnormal cell, such as neurologic diseases, central nervous system diseases, metabolic diseases, tumors (for example, malignant tumors (cancers)), infections, immune system diseases (for example, autoimmune diseases, allergic diseases, and inflammatory diseases), and proteinopathies. The pharmaceutical composition of the present invention may be directed, for example, to the treatment of a disease selected from the group consisting of cancers, autoimmune diseases, inflammatory diseases, and proteinopathies.

The malignant tumors (cancers) herein include, for example, leukemia, seminoma, melanoma, teratoma, lymphoma, neuroblastoma, neuroglioma, prostate cancer, uterine cancer, endometrial cancer, cervical cancer, ovarian cancer, adrenal cancer, thyroid cancer, skin cancer, head and neck cancer, digestive cancer, pancreatic cancer, breast cancer, and lung cancer, and metastases thereof.

For example, when a target disease is a cancer, an anticancer agent comprising the hairpin nucleic acid composition and/or the cell death-inducing composition of the present invention as active ingredients may be made by components in accordance with those of the pharmaceutical composition of the present aspect.

In such a case, the target cancer may be any cancer as described above. The target RNA may be an RNA expressed in a target cancer cell-specific manner and/or an RNA highly expressed in a target cancer cell-specific manner.

The anticancer agent of the present invention may optionally comprise another additional anticancer agent.

For example, when an inflammatory disease is a target disease, an anti-inflammatory agent comprising the hairpin nucleic acid composition and/or the cell death-inducing composition of the present invention as active ingredients may be made by components in accordance with those of the pharmaceutical composition of the present aspect.

In such a case, the target inflammatory disease may be any inflammatory disease as described in the section for the definitions. The target RNA may be an RNA expressed in a target inflammatory cell-specific manner and/or an RNA highly expressed in a target inflammatory cell-specific manner. For example, when a cell causing inflammatory reaction is present, the target RNA may be an RNA expressed and/or highly expressed in a manner specific to the cell causing the inflammatory reaction.

The anti-inflammatory agent of the present invention optionally comprises another additional anti-inflammatory agent.

3. Protein Function-Inhibiting Composition

3-1 Summary

A third aspect of the present invention is a protein function-inhibiting composition. The protein function-inhibiting composition of the present invention comprises the hairpin nucleic acid composition described in the first aspect A, as an active ingredient, and induces HCR in a site of interest comprising a target RNA. Use of the protein function-inhibiting composition of the present invention enables the function of a protein to be inhibited based on HCR, for example, in a cell of interest.

3-2 Components

The components of the protein function-inhibiting composition of the present aspect are described. The protein function-inhibiting composition of the present invention comprises an active ingredient as an essential component, and a carrier as an optionally selectable component. Each component will be specifically described below.

The protein function-inhibiting composition of the present invention comprises, as an essential active ingredient, an effective amount of the hairpin nucleic acid composition described in the first aspect A. The protein function-inhibiting composition preferably comprises an effective amount of the hairpin nucleic acid composition described in the first aspect A, comprising a protein-binding motif. Basically, the details of the active ingredient is in accordance with that of the pharmaceutical composition described in the second aspect except that a desired effect is inhibition of the function of a protein. Accordingly, differences are only described.

The protein function-inhibiting composition of the present invention is directed to the inhibition of the function of a protein. Accordingly, the protein function-inhibiting composition may comprise one or more active ingredients capable of inhibiting the function of a protein, as active ingredients, in addition to the hairpin nucleic acid composition described in the first aspect A. Specifically, the active ingredients include, for example, gene expression suppressing agents (for example, antisense nucleic acids, short hairpin nucleic acids, and the like), and function inhibitors at protein levels (for example, antibodies, aptamers, competitive inhibitors, and the like).

Basically, the details, such as a carrier and a dosage form, used in the protein function-inhibiting composition of the present invention are in accordance with those of the pharmaceutical composition of the second aspect. Accordingly, detailed descriptions thereof are omitted.

It is not necessary that the protein function-inhibiting composition of the present invention inhibits only a protein that directly binds to a protein-binding motif. For example, the protein function-inhibiting composition may inhibit a protein that indirectly binds to an HCR product through the protein that binds to a protein-binding motif.

4. Cell Death-Promoting Composition

4-1. Summary

A fourth aspect of the present invention is a cell death-promoting composition. The cell death-promoting composition of the present invention comprises the hairpin nucleic acid composition described in the first aspect A, as an active ingredient, and induces HCR in a cell of interest, comprising a target RNA. Use of the cell death-promoting composition of the present invention enables cell death to be promoted based on HCR in a cell of interest.

4-2. Components

The components of the cell death-promoting composition of the present aspect are described. The cell death-promoting composition of the present invention comprises an active ingredient as an essential component, and a carrier as an optionally selectable component. Each component will be specifically described below.

The cell death-promoting composition of the present invention comprises an effective amount of the hairpin nucleic acid composition described in the first aspect A, as an essential active ingredient. Basically, the details of the active ingredient is in accordance with that of the pharmaceutical composition described in the second aspect except that a desired effect is promotion of cell death. Accordingly, differences are only described.

The cell death-promoting composition of the present invention is targeted at promoting cell death. Accordingly, the cell death-promoting composition may comprise one or more active ingredients capable of promoting cell death, as active ingredients, in addition to the hairpin nucleic acid composition described in the first aspect A. Specifically, the active ingredients include, for example, compounds, drugs based on suppression of gene expression, and drugs based on inhibition of functions at protein levels, that induce cell death.

Basically, the details, such as a carrier and a dosage form, used in the cell death-promoting composition of the present invention are in accordance with those of the pharmaceutical composition of the second aspect. Accordingly, detailed descriptions thereof are omitted here.

It is not necessary that the cell death-promoting composition of the present invention induces cell death only in a cell comprising an HCR product. The cell death-promoting composition of the present invention can induce an immune response due to death of a cell comprising an HCR product, as described in Examples below. The immune response can secondarily promote cell death in a cell that does not have any HCR product but has similar abnormality. Herein, the promoted cell death is, for example, immunogenic cell death.

5. Nucleic Acid Immunity Induction Composition

5-1. Summary

A fifth aspect of the present invention is a nucleic acid immunity induction composition. The nucleic acid immunity induction composition of the present invention comprises the hairpin nucleic acid composition and/or the cell death-inducing composition described in the first aspect, as active ingredients, and induces HCR in a site of interest, comprising a target RNA. Use of the nucleic acid immunity induction composition of the present invention enables nucleic acid immunity to be induced based on HCR, for example, in a cell of interest.

5-2. Components

The components of the nucleic acid immunity induction composition of the present aspect are described. The nucleic acid immunity induction composition of the present invention comprises a hairpin nucleic acid composition and/or a cell death-inducing composition as essential components, which are active ingredients, and a carrier as an optionally selectable component. Each component is specifically described below.

The nucleic acid immunity induction composition of the present invention comprises, as essential active ingredients, effective amounts of the hairpin nucleic acid composition and/or the cell death-inducing composition described in the first aspect. Basically, the details of the active ingredients are in accordance with those of the pharmaceutical composition described in the second aspect except that a desired effect is nucleic acid immunity induction. Accordingly, differences are only described.

The nucleic acid immunity induction composition of the present invention is targeted at inducing nucleic acid immunity. Accordingly, the nucleic acid immunity induction composition may comprise one or more active ingredients capable of inducing nucleic acid immunity, as active ingredients, in addition to the hairpin nucleic acid composition and/or the cell death-inducing composition described in the first aspect. Specifically, the active ingredients include, for example, molecules that increase the activity of pattern-recognition receptors and/or downstream factors thereof (for example, agonists thereof, expression promoters, and the like), nucleic acid molecules that are recognized by other kinds of pattern-recognition receptors, and lipid molecules and sugar molecules that are recognized by pattern-recognition receptors that recognize anything other than nucleic acids.

Basically, the details, such as a carrier and a dosage form, used in the nucleic acid immunity induction composition of the present invention are in accordance with those of the pharmaceutical composition of the second aspect. Accordingly, detailed descriptions thereof are omitted.

The nucleic acid immunity induction composition of the present invention may induce nucleic acid immunity in a cell other than a cell comprising an HCR product. For example, when the hybridization chain structure is a structure that is recognized by cGAS, 2'-5'-cGAMP which is a second messenger synthesized by cGAS may migrate to adjacent cells and the like through gap junctions to also induce nucleic acid immunity in those cells.

Examples

<Example 1: Evaluation of HCR Efficiency in Cell-Free System>

(Purpose)

To examine a relationship between HCR efficiency and the structure of a hairpin nucleic acid in the experiment system of a cell-free system.

(Method)

1. Design of Hairpin Nucleic Acid

Sets of three kinds of hairpin nucleic acids in which miR-21 (SEQ ID NO: 1) was a target RNA were designed using online software NUPACK (http://www.nupack.org/). The sequence of each hairpin nucleic acid and the free energy change of the hairpin structure formation reaction thereof are set forth in Table 1.

[Table 1]

Table 1: Sequences of Hairpin Nucleic Acids used in Example 1

| Name of Nucleic Acid | Sequence | SEQ ID NO: | Free energy (kcal/mol) |
|---|---|---|---|
| HP1 (16) | <u>TCAACATC</u>**AGTCTGATAAGCTA***GG**GACTTT***CCTAGCTTATCAGACT** | 2 | -16.4 |
| HP2 (16) | **TAGCTTATCAGACT**GATGTTGA**AGTCTGATAAGCTA**<u>GG*AAAGTCCC*</u> | 3 | -12.8 |
| HP1 (15) | <u>TCAACATC</u>**AGTCTGATAAGCTA***GG**GACTTT***CCTAGCTTATCAGAC** | 4 | -15.3 |
| HP2 (15) | **TAGCTTATCAGACT**GATGTTG**AGTCTGATAAGCTA**<u>GG*AAAGTCCC*</u> | 5 | -12.6 |
| HP1 (13) | <u>TCAACATCAG</u>**TCTGATAAGCTA***GG**GACTTT***CCTAGCTTATCAGA** | 6 | -13.5 |
| HP2 (13) | **TAGCTTATCAGACT**GATGTTG**ATCTGATAAGCTA**<u>GG*AAAGTCCC*</u> | 7 | -10.0 |

Underline: protruding region, bold: stem region, italic: protein-binding motif

EP 4 382 110 A1

In the sets of the three hairpin nucleic acids (HP(16), HP(15), and HP(13)), the free energy changes of all the first hairpin nucleic acids (HP1) were the lowest. HP1(16), HP1(15), and HP1(13) were -16.4 kcal/mol, -15.3 kcal/mol, and -13.5 kcal/mol, respectively.

Each designed hairpin nucleic acid was chemically synthesized using a nucleic acid automatic synthesizer.

2. Evaluation of HCR Efficiency

For each set of the hairpin nucleic acids, miR-21 was added, at a final concentration of 0.1 $\mu$M, to a TE buffering solution comprising HP1 and a second hairpin nucleic acid (HP2), of which each of the concentrations was 1 $\mu$M, and the resultant liquid mixture was left at room temperature. A liquid mixture to which miR-21 was not added was used as control. The reaction solution was analyzed by 1% agarose gel electrophoresis after the incubation. SYBR™ Gold (Thermo Fisher Scientific) was used for staining the gel.

(Results)

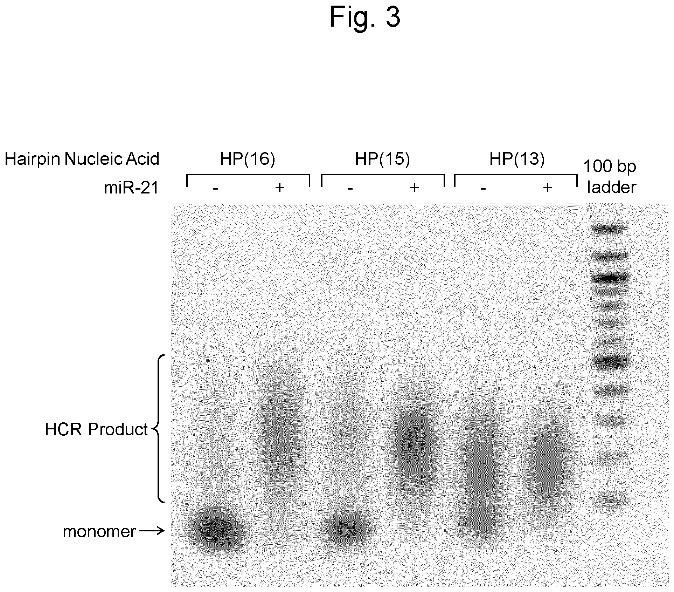

The results are indicated in Figure 3.

HCR was induced in both the three kinds of evaluated sets of the hairpin nucleic acids. In all the sets, a number of monomeric hairpin nucleic acid molecules existed when no miR-21 is added, and the monomers were prominently decreased by adding miR-21. In particular, in the set of the hairpin nucleic acids with HP1 having the free energy change of -16.4 kcal/mol and -15.3 kcal/mol, an HCR product was hardly observed when no miR-21 is added, and a reaction was found to proceed in a manner dependent on miR-21 which was a target RNA.

The set of the hairpin nucleic acids HP(16) with the highest HCR efficiency was used in the following experiment.

<Example 2: Evaluation of Nuclease Resistance of Hairpin Nucleic Acid>

(Purpose)

A relationship between nuclease resistance and the length of the protruding region of a hairpin nucleic acid was examined.

(Method)

HP1(16) or HP2(16) were added, at a final concentration of 0.5 $\mu$M, to a DMEM solution (Gibco) containing 10% fetal bovine serum (FBS; Biowest) and 0.5% penicillin-streptomycin (NACALAI TESQUE, INC.), and the resultant was left at 37°C. An internucleotide bond in the protruding regions of HP1(16) and HP2(16) used was subjected to phosphorothioate modification. Part of the reaction liquid (20 $\mu$L) was collected at certain time intervals, and formamide (10 $\mu$L) was added thereto to stop the reaction. Then, the resultant was analyzed by 5% polyacrylamide gel electrophoresis. SYBR™ Gold (Thermo Fisher Scientific) was used for staining the gel.

(Results)

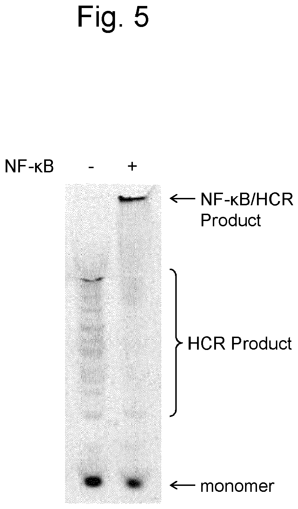

The results are indicated in Figure 4.

It was revealed that a sufficient amount of each of the hairpin nucleic acids remained for around several hours even in the presence of FBS comprising a large amount of nuclease. Since most of each of the hairpin nucleic acids underwent nuclease digestion after a lapse of one day, it was revealed that the unreacted hairpin nucleic acids were digested and appropriately removed by nuclease. HP1(16) and HP2(16) had protruding regions of 8 bases and 10 bases, respectively, but major difference was not observed in their nuclease resistances. Therefore, nuclease activity was found not to greatly depend on the length of a protruding region.

<Example 3: Evaluation of Efficiency of Capturing Protein of HCR Product>

(Purpose)

The efficiency of capturing the protein of a protein-binding motif on an HCR product was examined.

(Method)

Each of HP1(16) and HP2(16) has the recognition sequence of NF-$\kappa$B. Thus, miR-21 was added to a TE buffering solution comprising HP1(16) with fluorescence modification by FAM (FAM-HP 1 (16); Table 2, SEQ ID NO:8) and HP2(16), of which each of the concentrations was 0.1 μM, at a final concentration of 10 nM, and the resultant was left at room temperature. Recombinant NF-κB (Cayman Chemical) was added to the reaction solution at a final concentration of 50 ng/μL, and the resultant was left to stand. A reaction solution to which NF-κB was not added was used as control. The binding of NF-κB was detected using a gel shift assay. First, the reaction solution was subjected to 5% polyacrylamide gel electrophoresis. Bands were detected by detecting the fluorescence of FAM using Gel Doc™ EZ Imager (BioRad; excitation wavelength of 430 nm to 460 nm).

The sequences of hairpin nucleic acids used in Examples 3 and 4 are shown below. As shown in Table 2 below, in FAM-HP1(16) (SEQ ID NO:8), internucleoside bonds from the first base to the ninth base from the 5' end are phosphorothioate bonds, and the 21st nucleotide from the 5' end is a thymidine analog dT-FAM (3'). In TAMRA-HP2(16) (SEQ ID NO:9), internucleoside bonds from the 36th base to the 46th base from the 5' end are phosphorothioate bonds, and the 46th nucleotide from the 5' end is cytidine to which 5-TAMRA bound through a C6 amino linker (catalog number: 26-6418, Gene Link).

[Table 2]

Table 2: Sequence of Hairpin Nucleic Acids used in Examples 3 and 4

| Name of Nucleic Acid | Sequence (5' → 3') | SEQ ID NO: |
|---|---|---|
| FAM-HP1 (16) | T^C^A^A^C^A^T^C^AGTCTGATAAGCT*AGGGACTTTCCTAGCTTATCAGACT | 8 |
| TAMRA-HP2 (16) | TAGCTTATCAGACTGATGTTGAAGTCTGATAAGCTA^G^G^A^A^A^G^T^C^C^C** | 9 |
| ^: Phosphorothioate, *: FAM-T, **: C-TAMRA | | |

(Results)

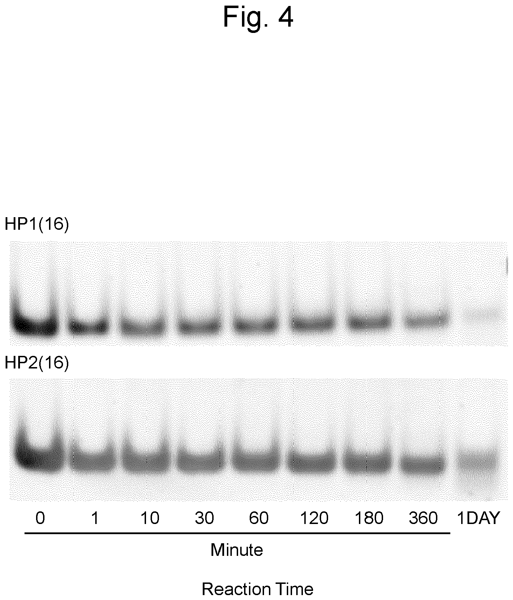

The results are indicated in Figure 5.

When no NF-κB is added, HCR products were found to be normally generated. Nucleic acid chains having various lengths were detected as the HCR products (arrowheads in Figure 5). When NF-κB is added, bands of sole HCR products were hardly detected, and bands with very low mobility were observed. Thus, a complex of the HCR product and NF-κB (NF-κB/HCR product) was found to be formed with high efficiency regardless of the length of an HCR product.

<Example 4: Evaluation of HCR Efficiency in Cell Line>

(Purpose)

The occurrence of HCR in human cells was confirmed, and the efficiency thereof was examined.

(Method)

In glass bottom dishes of 3.5 cm, $3.0 \times 10^4$ HEK293T cells and HeLa cells were seeded, respectively, and cultured in 200 μL of DMEM solutions containing 10% FBS and 0.5% penicillin-streptomycin until reaching about 90% confluency. A TE buffering solution comprising FAM-HP1(16) and HP2(16) with fluorescence modification by TAMRA (TAMRA-HP2(16); Table 2, SEQ ID NO:9) at a concentration of 0.1 μg/μL each, was microinjected into each of HEK293T and HeLa cells by Femtotips (Eppendorf). Fluorescence resonance energy transfer (FRET) reaction occurring when HCR occurred to allow FAM and TAMRA to be close to each other was detected by observing the cells with a confocal microscope. A FRET efficiency was calculated as a ratio between fluorescence intensities at 521 nm and 575 nm when excited at 488 nm. The relative value of the FRET efficiency at each time point with the FRET efficiency at the time of start of the reaction set to 1, was calculated as a relative FRET efficiency. The experiment was repeated four times.

(Results)

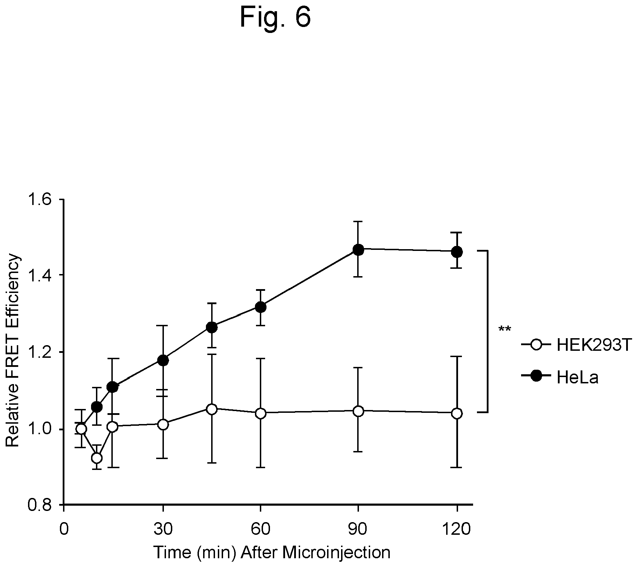

The results are indicated in Figure 6.

No change in FRET efficiency was observed in the human embryonic kidney HEK293T cells, which express little miR-21 (white circle graph in Figure 6). In contrast, an increase in FRET signal over time was observed as a result of introducing hairpin nucleic acids into the human cervical cancer HeLa cells, which express miR-21 (black circle graph in Figure 6). Based on the results above, HP(16) designed herein was found to induce HCR in a manner specific to cells expressing miR-21. Moreover, HCR was found to start simultaneously with the introduction of the hairpin nucleic acids into the cells, and to be completed within about 90 minutes.

<Example 5: Evaluation of Efficiency of Promotion of Cell Death by HCR>

(Purpose)

Cell death was confirmed to be promoted by HCR in human cells, and the efficiency thereof was examined.

(Method)

In 24-well multi-well plates, $1.0 \times 10^5$ HEK293T cells and HeLa cells were seeded, respectively, and cultured in 500 μL of DMEM solution containing 10% FBS and 0.5% penicillin-streptomycin until reaching about 90% confluency. Lipofectamine LTX (Thermo Fisher Scientific) and 50 μL of OPTI-MEM were added to a mixed solution of 50 μL OPTI-MEM (Thermo Fisher Scientific) and 1 μL of Lipofectamine Plus Reagent (Thermo Fisher Scientific), comprising HP1(16) and HP2(16) at concentrations of 0 μg/μL, 0.5 μg/μL, and 1 μg/μL, and the resultant was left at room temperature. Then, the reaction solution was added to a dish. Further thereafter, medium was replaced with 500 μL of DMEM solution containing 10% FBS and 0.5% penicillin-streptomycin. 24 hours later, medium was replaced with 400 μL of DMEM solution containing 9% FBS, 0.45% penicillin-streptomycin, and 10% PrestoBlue (Invitrogen). Cell death was detected by measuring the fluorescence of PrestoBlue. For each cell type, a relative cell survival rate was calculated as a relative value with a cell survival rate under a condition without hairpin nucleic acid set to 100%. The experiment was repeated four times.

(Results)

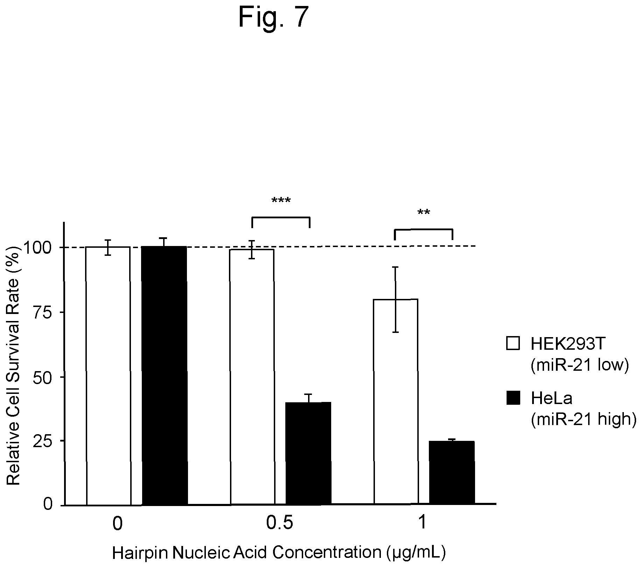

The results are indicated in Figure 7.

In the HEK293T cells, which express little miR-21, a significant decrease in cell survival rate was not observed even when the concentration of hairpin nucleic acid was increased (white bar graph in Figure 7). In contrast, it was found that in the HeLa cells, which express miR-21, a cell survival rate was prominently decreased by introducing a hairpin nucleic acid, and cell death was induced depending on the amount of the introduced hairpin nucleic acid (black bar graph in Figure 7). Based on the above, the introduction of the hairpin nucleic acid was found to promote cell death through HCR in a manner specific to cells in which a target RNA existed. Moreover, observation with a fluorescence microscope revealed that the introduction of the hairpin nucleic acid caused formation of an aggregate and induction of liquid-liquid phase separation.

Cell death is known not to be induced by simple induction of HCR. Therefore, it was suggested that the aggregate was formed by capturing NF-κB, whereby the cell death was promoted.

<Example 6: Evaluation of Efficiency of Induction of Immune Response by HCR>

(Purpose)

An immune response was confirmed to be induced by HCR in human cells, and the efficiency thereof was examined.

(Method)

Lipofection was performed according to Example 5 except that MCF7 cells were used as cultured cells. 5 μL of medium was collected 24 hours after medium replacement following the lipofection, and IFN-β was quantified using ELIZA (PBL Assay Science). The experiment was performed under a condition, without hairpin nucleic acid HP(16), as negative control (NC), while the experiment was performed under a condition, in which poly(dG:dC) (InvivoGen) was used instead of HP(16), as positive control (PC). The amount of relative IFN-β was calculated as a relative value with the amount of IFN-β of NC set to 1.

(Results)

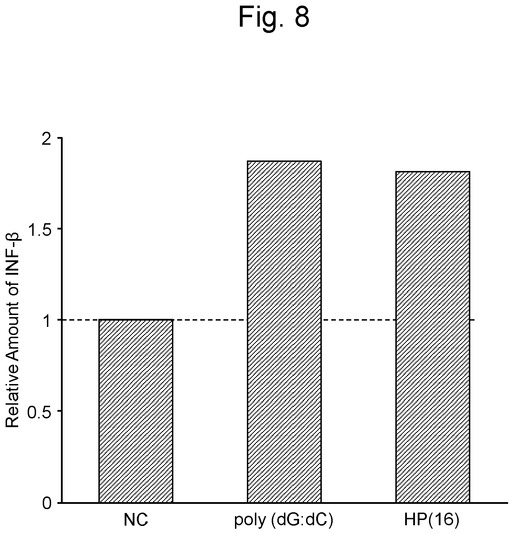

The results are indicated in Figure 8.

In PC in which poly(dG:dC), which is known to have a strong immunostimulation ability, was introduced into human breast cancer MCF7 cells, which express miR-21, INF-β at a level about twice as much as that in NC was produced. When HP(16) was introduced into the same cells, INF-β at a high level equivalent to that in PC was observed. Based on the above, an HCR product was found to induce nucleic acid immunity.

Commonly, a cancer cell is known to be protected from an immune response by NF-κB. Based on the above, it was shown that the hairpin nucleic acid HP(16) induced cell death through HCR in a manner specific to a cell expressing miR-21, which is a target RNA, by both the inhibition of the function of NF-κB by capturing NF-κB and the induction of nucleic acid immunity.

<Example 7: Evaluation of HCR Efficiency according to Cell Type>

(Purpose)

A relationship between the cell types and the efficiency of occurrence of HCR was examined.

(Method)

The introduction of HP(16) into cells and the evaluation of HCR efficiency were performed in a procedure in accordance with that in Example 4 except for cells used. Human breast cancer MDA-MB-231 cells and human alveolar basement epithelial glandular cancer A549 cells were used as the cells into HP(16) was introduced, in addition to human embryonic kidney HEK293T cells and human cervical cancer HeLa cells. As in Example 4, a relative FRET efficiency was calculated as a ratio between fluorescence intensities at 521 nm and 575 nm in the case of excitement at 488 nm.

In 24-well multi-well plates, $5.0 \times 10^4$ HEK293T cells, HeLa cells, MDA-MB-231 cells, and A549 cells were seeded, respectively, and cultured in 500 μL of Dulbecco's modified Eagle's medium (DMEM) solution containing 10% FBS and 0.5% penicillin-streptomycin. After 24-hour culture, medium was replaced with Opti-MEM (Invitrogen).

Each hairpin nucleic acid in an amount of 0.5 μg per well was introduced into each cell using Lipofectamine LTX (Invitrogen). As the hairpin nucleic acids for the introduction, a set (HP(16)) of HP1(16) with fluorescence modification by Cy5 (Cy5-HP1(16); Table 3, SEQ ID NO:10) and HP2(16) with fluorescence modification by FAM (FAM-HP2(16); Table 3, SEQ ID NO:11), or a set of hairpin nucleic acids having scrambled sequences (scrambled hairpin nucleic acids) was used.

After 3-hour culture, FRET reaction occurring when HCR occurred to allow FAM and Cy5 to be close to each other was observed. The observation was performed by observing the cells with a confocal microscope. A FRET efficiency (relative value) indicates a FRET signal intensity when HP(16) was introduced relative to a FRET signal intensity when a scrambled hairpin nucleic acid was introduced. The FRET signal intensity under each condition was calculated as a ratio of the fluorescence intensity of Cy5 over the fluorescence intensity of FAM observed when excited at 488 nm.

The experiment was repeated four times.

Statistical significance was determined by unpaired t test.

The sequences of the hairpin nucleic acids used in Example 7 is shown below. As set forth in Table 3 below, in Cy5-HP1(16) (SEQ ID NO:10), internucleoside bonds from the first base to the ninth base from the 5' end are phosphorothioate bonds, and the 46th nucleotide from the 5' end is thymidine to which Cy5 bound through a C6 amino linker as that used in Example 4. In FAM-HP2(16) (SEQ ID NO:11), internucleoside bonds from the 36th base to the 46th base from the 5' end are phosphorothioate bonds, and the 21st nucleotide from the 5' end is a thymidine analog dT-FAM (3').

[Table 3]

Table 3: Sequences of Hairpin Nucleic Acids used in Example 7

| Name of Nucleic Acid | Sequence (5' → 3') | SE Q ID NO : |
|---|---|---|
| Cy5-HP1 (16) | T^C^A^C^A^T^C^AGTCTGATAAGCTAGGGACTTTCCTAGCTTATCAGACT** | 10 |
| FAM-HP2 (16) | TAGCTTATCAGACTGATGTTGAAGT*CTGATAAGCTA^G^G^A^A^A^A^ G^T^C^C^C | 11 |
| ^: Phosphorothioate, *: FAM-T, **: Cy5-T | | |

(Results)

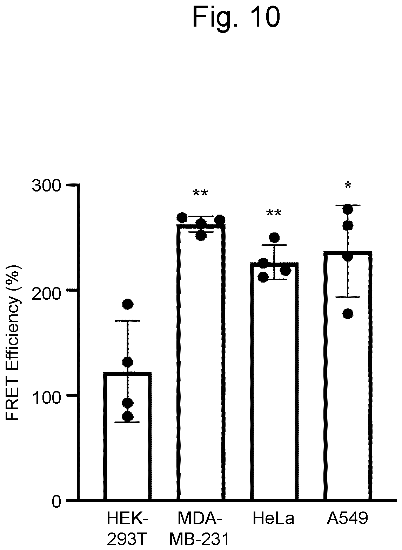

The results are indicated in Figure 10.

In human embryonic kidney HEK293T cells, which express little miR-21, a FRET signal intensity was equivalent (around 100%) to that when the scrambled hairpin nucleic acids were used (Figure 10). This agrees with the results of Example 4 (white circle graph in Figure 6). In contrast, an increase in FRET signal intensity was observed in each of human cervical cancer HeLa cells, human breast cancer MDA-MB-231 cells, and human alveolar basement epithelial glandular cancer A549 cells, which express miR-21 (Figure 10).

Based on the results above, HP(16) designed herein was found to induce HCR in a manner specific to cells expressing miR-21. Further, cells expressing miR-21 were found to induce HCR regardless of the kind of a cancer.

<Example 8: Evaluation of Efficiency of Promotion of Cell Death according to Cell Types>

(Purpose)

Influences of the cell types on the efficiency of promoting cell death by HCR were examined.

(Method)

In 24-well multi-well plates, $5.0 \times 10^4$ A549 cells, MDA-MB-231 cells, and B16 cells were seeded, respectively, and cultured in 500 $\mu$L of DMEM solution containing 10% FBS and 0.5% penicillin-streptomycin until reaching about 80% confluency. Then, medium was replaced with OPTI-MEM.

HP(16) in an amount of 0.5 $\mu$g per well was introduced into each cell using Lipofectamine LTX (Invitrogen).

48 hours later, the medium was replaced with a standard growth solution. Cell death was detected by measuring the fluorescence of PrestoBlue (Invitrogen). As in Example 5, the fluorescence was measured using a multi-well Cytation™ 5 plate reader (BioTek Instruments).

For each cell type, a relative cell survival rate was calculated as relative value with a cell survival rate under a condition without hairpin nucleic acid set to 100%.

The experiment was repeated four times.

Statistical significance was determined by unpaired t test.

(Results)

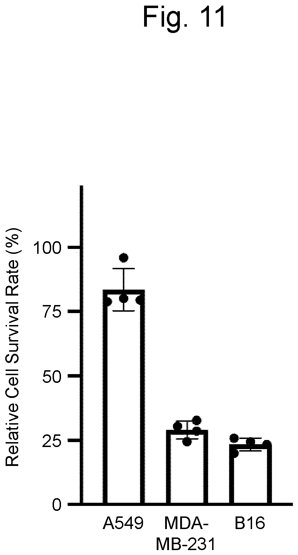

The results are indicated in Figure 11.

In MDA-MB-231 cells and mouse malignant melanoma B16 cells, the cell survival rate was prominently decreased by introducing HP(16) (Figure 11). In contrast, a major decrease in cell survival rate by introducing HP(16) was not observed in A549 cells.

All the A549, MDA-MB-231, and B16 cells express miR-21. Nevertheless, since the results of the cell survival rates varied depending on the cell types, it was suggested that there was a factor influencing the efficiency of promoting cell death, in addition to the presence or absence of the expression of miR-21.

<Example 9: Evaluation of Influence of cGAS-STING Pathway on Efficiency of Promotion of Cell Death>

(Purpose)

The influence of a cGAS-STING pathway on the efficiency of promoting cell death by HCR was examined.

(Method)

In a 6-well multi-well plate, $5.0 \times 10^5$ HeLa cells were seeded, and cultured in 500 $\mu$L of DMEM solution containing 10% FBS and 0.5% penicillin-streptomycin until reaching about 80% confluency. Then, medium was replaced with OPTI-MEM.

STING siRNA (human TMEM173 siRNA, catalog number: AM16708; Silencer select siRNA, Thermo Fisher Scientific) was added into the medium so as to achieve a final concentration of 5 nM, and the resultant was introduced into each cell.

24 hours later, medium was replaced with a standard growth solution. After further 24-hour culture, the cells were seeded in a 24-well multi-well plate, and cell death was detected. The cell death was detected basically in a manner as in Example 8. However, as the cells, HeLa cells into which STING siRNA was introduced or was not introduced were used. HP(16) or poly(dA:dT) (Invitrogen) was used as a nucleic acid to be introduced.

The experiment was repeated four times.

Statistical significance was determined by unpaired t test.

(Results)

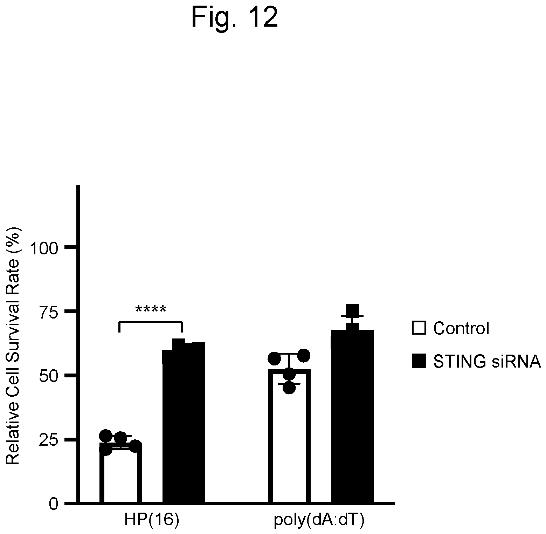

The results are indicated in Figure 12.

In the HeLa cells into which STING siRNA was not introduced, a cell survival rate was more greatly decreased by HP(16) than when poly(dA:dT) known to have a strong immunostimulation ability was introduced (Figure 12). Based on the above, HP(16) was found to be able to induce cell death more efficiently than poly(dA:dT).

In contrast, the induction of the cell death by HP(16) was prominently suppressed in the HeLa cells into which STING siRNA was introduced (Figure 12).

Based on the above, HP(16) was found to induce cell death through a cGAS-STING pathway. This agrees with the fact that cell death by HP(16) was not induced in A549 cells known to express no STING protein (Figure 11).

<Example 10: Evaluation of Efficiency of Induction of Immune Response by HCR>

(Purpose)

The efficiency of inducing an immune response by HCR in human cells was examined.

(Method)

Cell culture and lipofection were performed according to Example 9 except that HP(16) was used as a nucleic acid to be introduced. Cells were suspended 18 hours after medium replacement following the lipofection, and RNA was extracted using Nucleo Spin RNA XS (Macherey-Nagel). The extracted RNA was analyzed by qRT-PCR using One Step TB Green PrimeScript PLUS RT-PCR Kit (TaKaRa Bio) and Rotor-Gene 3000 (QIAGEN). The following primers were used as primers for mRNA for IFN-$\beta$.

Forward primer: 5'-ACAGGTTACCTCCGAAACTGAAGA-3' (SEQ ID NO:12)
Reverse primer: 5'-TTAGCCATCAGTCACTTAAACAGCA-3' (SEQ ID NO:13)

The relative amount of IFN-$\beta$ mRNA was calculated as the relative value of the standardized amount of IFN-$\beta$ mRNA under each condition standardized with the standardized amount of IFN-$\beta$ mRNA of a control group, which was standardized with the amount of GAPDH mRNA, set to 1.

Statistical significance was determined by unpaired t test.

(Results)

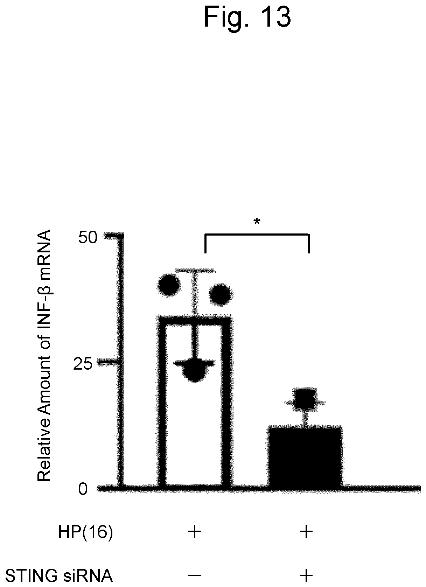

The results are indicated in Figure 13.

When no HP(16) was introduced, mRNA of INF-$\beta$ was hardly detected from HeLa cells (data was not shown). As in the results in Example 6, mRNA of INF-$\beta$ was greatly increased by introducing HP(16) (Figure 13). However, the increase was prominently suppressed in the HeLa cells into which STING siRNA was introduced (Figure 13).

Based on the above, the induction of nucleic acid immunity by an HCR product was found to occur through a cGAS-STING pathway.

<Example 11: Evaluation of Efficiency of Inducing Cell Death against *In Vivo* Cancer Cells>

(Purpose)

The efficiency of inducing cell death against *in vivo* cancer cells by HCR was examined.

(Method)

First, mice were allowed to carry cancer cells. By subcutaneous injection, $1.5 \times 10^5$ B16 cells suspended in 100 $\mu$L of PBS were administered to 6-week-old C57BL/6 mice. Then, mice reaching a tumor volume of 100 mm$^3$ were subjected to a medication experiment.

The medication was performed 3 times every 4 days by peritumoral administration. As a drug, 8.4 $\mu$g of HP(16)

or poly(dA:dT) was administered using AteloGene Local Use Quick Gelation (KOKEN). PBS was administered to a control group.

Tumor volumes and body weights were measured 0 days after, 4 days after, 6 days after, 8 days after, 9 days after, 12 days after, and 15 days after the beginning of the administration.

Experiments were performed with approval by the Ethical Review Board in the University of Tokyo.

A mouse was euthanized in the stage of achieving a tumor volume of 2000 mm$^3$ or more as an end point. The tumor volume was calculated based on the following equation:

$$(\text{Tumor volume}) = (\text{major diameter of tumor}) \times (\text{minor diameter of tumor})^2/2$$

(Results)

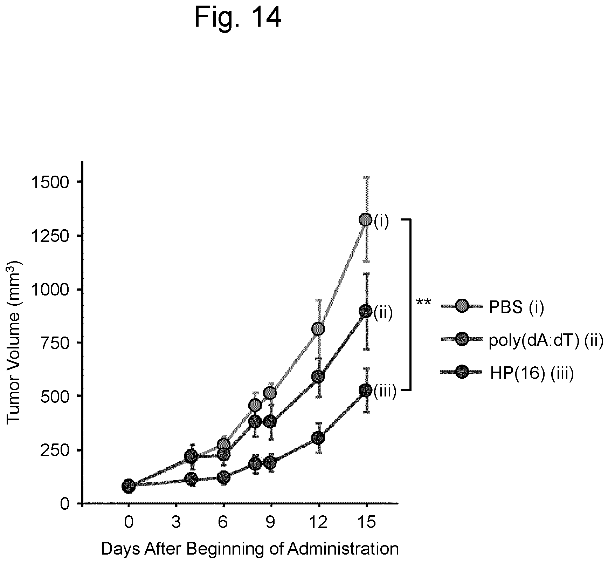

The results are indicated in Figure 14.

In a poly(dA:dT) administration group, suppression of an increase in tumor volume was observed following 6 days after the beginning of the administration, in compared to the control group to which PBS was administered ((i) and (ii) in Figure 14). In contrast, from the first time point of measurement after the beginning of the administration, an HP(16) administration group exhibited prominent suppression of an increase in tumor volume in comparison with the control group and the poly(dA:dT) administration group, and 15 days after the beginning of the administration, the tumor volume in the HP(16) administration group was significantly small compared to the control group ((iii) in Figure 14). The body weights of the mice were not substantially changed in all the groups.

Based on the above, the induction of cell death of tumor cells by an HCR product was found to act also effectively *in vivo* and to be useful for treatment of cancers.

All publications, patents, and patent application cited in the present specification are incorporated herein by reference in their entirety.

**Claims**

1. A cell death-inducing composition comprising a hairpin nucleic acid having a hairpin structure capable of forming a hybridization chain structure, wherein the cell death-inducing composition comprises a starting hairpin nucleic acid that hybridizes with a target RNA; and an elongating hairpin nucleic acid that hybridizes with the starting hairpin nucleic acid or another elongating hairpin nucleic acid.

2. The cell death-inducing composition according to claim 1, wherein

   the starting hairpin nucleic acid comprises a target RNA-binding cassette that hybridizes with a target RNA and an elongating hairpin nucleic acid-binding cassette that hybridizes with the elongating hairpin nucleic acid, and the elongating hairpin nucleic acid comprises a protruding region cassette that hybridizes with the starting hairpin nucleic acid or another elongating hairpin nucleic acid, and a non-protruding region cassette that hybridizes with further another elongating hairpin nucleic acid.

3. The cell death-inducing composition according to claim 1 or 2, wherein the target RNA binds to the target RNA-binding cassette to allow a hairpin structure of the starting hairpin nucleic acid to be dissociated to enable hybridization between the elongating hairpin nucleic acid-binding cassette and the protruding region cassette of the elongating hairpin nucleic acid, and the hybridization allows a hairpin structure of the elongating hairpin nucleic acid to be dissociated to enable hybridization between the non-protruding region cassette and the protruding region cassette of another elongating hairpin nucleic acid to form a straight-chain double-stranded nucleic acid having the hybridization chain structure.

4. The cell death-inducing composition according to any one of claims 1 to 3, wherein the elongating hairpin nucleic acid comprises hairpin nucleic acids of 5'-end protruding type and 3'-end protruding type.

5. The cell death-inducing composition according to any one of claims 1 to 4, wherein the target RNA-binding cassette is capable of further hybridizing with a non-protruding region cassette of the elongating hairpin nucleic acid.

6. The cell death-inducing composition according to any one of claims 1 to 5, wherein the starting hairpin nucleic acid and/or elongating hairpin nucleic acid further comprise a whole or part of a protein-binding motif.

7. The cell death-inducing composition according to claim 6, wherein

the loop region and/or the protruding region comprise a whole or part of the protein-binding motif; and
the whole of the protein-binding motif comprises a double strand when the hybridization chain structure is formed.

8. The cell death-inducing composition according to any one of claims 1 to 7, wherein for the starting hairpin nucleic acid, a free energy change of hairpin structure formation reaction is -20 to -10 kcal/mol.

9. The cell death-inducing composition according to any one of claims 1 to 8, wherein the hairpin nucleic acid comprises DNA and/or RNA nucleotides.

10. The cell death-inducing composition according to claim 9, wherein the nucleotides comprise a modified nucleotide.

11. The cell death-inducing composition according to any one of claims 1 to 10, wherein the target RNA is mRNA or miRNA.

12. The cell death-inducing composition according to any one of claims 1 to 11, wherein the target RNA is RNA that is expressed or highly expressed in a cell-specific manner.

13. A pharmaceutical composition comprising the cell death-inducing composition according to any one of claims 1 to 12 as an active ingredient.

14. The pharmaceutical composition according to claim 13, wherein the pharmaceutical composition is for treating a disease selected from a group consisting of cancers, immune system diseases, and neurodegenerative diseases.

15. An anticancer agent comprising a hairpin nucleic acid having a hairpin structure capable of forming a hybridization chain structure, wherein the anticancer agent comprises a starting hairpin nucleic acid that hybridizes with a target RNA, and an elongating hairpin nucleic acid that hybridizes with the starting hairpin nucleic acid or another elongating hairpin nucleic acid.

16. An anti-inflammatory agent comprising a hairpin nucleic acid having a hairpin structure capable of forming a hybridization chain structure, wherein the anti-inflammatory agent comprises a starting hairpin nucleic acid that hybridizes with a target RNA, and an elongating hairpin nucleic acid that hybridizes with the starting hairpin nucleic acid or another elongating hairpin nucleic acid.

17. A composition comprising a starting hairpin nucleic acid and an elongating hairpin nucleic acid having a hairpin structure capable of forming a hybridization chain structure, wherein

the starting hairpin nucleic acid comprises a target RNA-binding cassette that hybridizes with a target RNA, and an elongating hairpin nucleic acid-binding cassette that hybridizes with the elongating hairpin nucleic acid;
the elongating hairpin nucleic acid comprises a protruding region cassette that hybridizes with the starting hairpin nucleic acid or another elongating hairpin nucleic acid, and a non-protruding region cassette that hybridizes with further another elongating hairpin nucleic acid; and
the starting hairpin nucleic acid and/or the elongating hairpin nucleic acid further comprise a whole or part of a protein-binding motif.

Fig. 1

A

B

(I)               (II)

C

Elongating Hairpin Nucleic Acid (I)

protruding region cassette      non-protruding region cassette

a        b        c        b'

Target RNA

a        b        c        b'        a        b

Target RNA-binding cassette      elongating hairpin nucleic acid-binding cassette      protruding region cassette

Starting Hairpin Nucleic Acid      Elongating Hairpin Nucleic Acid (II)

EP 4 382 110 A1

Fig. 2

A

STEP 1                          STEP 2                          STEP 3

B

EP 4 382 110 A1

# Fig. 3

# Fig. 4

HP1(16)

HP2(16)

| 0 | 1 | 10 | 30 | 60 | 120 | 180 | 360 | 1DAY |

Minute

Reaction Time

# Fig. 5

NF-κB    -    +

← NF-κB/HCR Product

} HCR Product

← monomer

# Fig. 6

# Fig. 7

# Fig. 8

Fig. 9

A

       

B

HCR Product

EP 4 382 110 A1

Fig. 10

# Fig. 11

# Fig. 12

# Fig. 13

# Fig. 14

## INTERNATIONAL SEARCH REPORT

| International application No. |
| --- |
| **PCT/JP2022/026323** |

**A. CLASSIFICATION OF SUBJECT MATTER**

*A61K 31/713*(2006.01)i; *A61P 25/28*(2006.01)i; *A61P 35/00*(2006.01)i; *A61P 37/02*(2006.01)i; *C12N 15/11*(2006.01)i
FI:    A61K31/713; A61P25/28; A61P35/00; A61P37/02; C12N15/11 Z ZNA

According to International Patent Classification (IPC) or to both national classification and IPC

**B. FIELDS SEARCHED**

Minimum documentation searched (classification system followed by classification symbols)

A61K31/713; A61P25/28; A61P35/00; A61P37/02; C12N15/11

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | US 2007/0087334 A1 (DIRKS, Robert. PIERCE, Niles A.) 19 April 2007 (2007-04-19)<br>claims 17-18, paragraph [0036], examples 1, 3 | 1-15, 17 |
| X | VENKATARAMAN, Suvir et al. Selective cell death mediated by small conditional RNAs. PNAS. 07 September 2010, vol. 107, pp. 16777-16782, supporting information, <DOI: 10.1073/pnas.1006377107><br>abstract, fig. 1, supporting information | 1-9, 11-15, 17 |
| A | DIRKS, Robert M. et al. Retraction. PNAS. 17 December 2012, vol. 110, p. 384, <DOI: 10.1073/pnas.1221212110><br>entire text | 1-17 |
| X | PATEL, Kalpesh. KERN, Scott E. "Selective cell death mediated by small conditional RNAs" is not selective. Cancer Biology & Therapy. 31 May 2013, vol. 14, pp. 693-696, <DOI: 10.4161/cbt.25093><br>abstract, pp. 695-696 | 1-9, 11-15, 17 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **08 September 2022** | **20 September 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
|---|
| **PCT/JP2022/026323** |

**C. DOCUMENTS CONSIDERED TO BE RELEVANT**

| Category* | Citation of document, with indication, where appropriate, of the relevant passages | Relevant to claim No. |
|---|---|---|
| Y | 森廣 邦彦, DNAナノテクノロジーを駆使したマイクロRNA応答型デコイ核酸医薬の創製, 倉田奨励金研究報告書 [online], vol. 49, receipt no.1384, 01 September 2020, [retrieved on 05 September 2022], Retrieved from the Internet: <URL: https://www.hitachi-zaidan.org/activities/kurata/reseach-report49.html> entire text, (MORIHIRO, Kunihiko), non-official translation (Creation of microRNA-responsive decoy type nucleic acid drugs using DNA nanotechnology. Research Report of The Kurata Grants [online].) | 1-17 |
| Y | 森廣 邦彦, タンデムリピート長鎖DNAの細胞内化学構築, ACT-X 年次報告書 [online], 研究領域「生命と化学」 2019年度研究年報, 2020, [retrieved on 05 September 2022], Retrieved from the Internet: <URL: https://www.jst.go.jp/kisoken/act-x/evaluation/nenpou/r01/r01_02.html> entire text, (MORIHIRO, Kunihiko), non-official translation (Intracellular chemical construction of tandem repeated-long chain DNA. ACT-X Annual Report [online]. Research Area "Life and Chemistry" Annual Research Report 2019.) | 1-17 |
| Y | US 2009/0227661 A1 (WANG, Zhiguo) 10 September 2009 (2009-09-10) examples | 1-17 |
| Y | 大住 拓輝, Multiple decoy construction system responding to microRNA. (マイクロRNA応答型デコイ核酸多重構築システム), 第20回東京大学生命科学シンポジウムポスター発表の要旨 [online], 21 October 2020, [retrieved on 17 March 2021], Retrieved from the Internet: <URL: http://www.todaibio.info/> entire text, non-official translation (OSUMI, Hiroki. Abstracts of 20th Life Science Symposium Poster Presentation of University of Tokyo [online].) | 1-17 |

Form PCT/ISA/210 (second sheet) (January 2015)

INTERNATIONAL SEARCH REPORT
Information on patent family members

International application No.

**PCT/JP2022/026323**

| Patent document cited in search report | Publication date (day/month/year) | Patent family member(s) | Publication date (day/month/year) |
|---|---|---|---|
| US 2007/0087334 A1 | 19 April 2007 | WO 2007/044727 A2 | |
| | | EP 1931806 A2 | |
| US 2009/0227661 A1 | 10 September 2009 | WO 2007/071069 A1 | |

Form PCT/ISA/210 (patent family annex) (January 2015)

49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021128492 A

- WO 2007143315 A

**Non-patent literature cited in the description**

- Approved Oligonucleotide Therapeutics. Division of Molecular Target and Gene Therapy Products, National Institute of Health Sciences, May 2021

- **SHEN, W. et al.** *Nat. Biotechnol.,* 2019, vol. 37, 640-650
- **ALTSCHUL, S. F. et al.** *J. Mol. Biol.,* 1990, vol. 215, 403-410